(19) Europäisches Patentamt
European Patent Office
Office européen des brevets

(11) **EP 3 949 842 A1**

(12) **EUROPEAN PATENT APPLICATION**
published in accordance with Art. 153(4) EPC

(43) Date of publication:
**09.02.2022 Bulletin 2022/06**

(21) Application number: **20778070.1**

(22) Date of filing: **17.03.2020**

(51) International Patent Classification (IPC):
**A61B 5/0285** (2006.01)

(52) Cooperative Patent Classification (CPC):
**A61B 5/0285**

(86) International application number:
**PCT/JP2020/011711**

(87) International publication number:
**WO 2020/196093 (01.10.2020 Gazette 2020/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.03.2019 JP 2019062459**

(71) Applicant: **Sony Group Corporation
Tokyo 108-0075 (JP)**

(72) Inventors:
• **KAWAMOTO, Yohei
Tokyo 108-0075 (JP)**
• **OGAWA, Sayaka
Tokyo 108-0075 (JP)**
• **WAKITA, Yoshihiro
Tokyo 108-0075 (JP)**

(74) Representative: **MFG Patentanwälte
Meyer-Wildhagen Meggle-Freund
Gerhard PartG mbB
Amalienstraße 62
80799 München (DE)**

(54) **INFORMATION PROCESSING DEVICE, INFORMATION PROCESSING METHOD, AND PROGRAM**

(57) An information processing device including a physiological condition estimator (123) configured to estimate a physiological condition of a user who is in a given physical state based on a first blood flow amount change of the user and a second blood flow amount change of the user or another user in the given physical state, the first blood flow amount change and the second blood flow amount change being acquired by a blood flow information detector, is provided.

FIG.3

**Description**

Field

The present disclosure relates to an information processing device, an information processing method and a program.

Background

In recent years, there has been a progress in development of techniques enabling casual measurement of an amount of blood flow, a rate of blood flow, etc., that are information on a blood flow of a user (referred to as blood flow information below). It is possible to determine a physiological condition (such as dehydration) of a user based on an amount of blood flow that is measured by such a technique. For example, the technique disclosed in Patent Literature 1 below can be taken as an example of such a technique.

Citation List

Patent Literature

Patent Literature 1: Japanese Laid-open Patent Publication No. 2015-54219

Summary

Technical Problem

While casual measurement of blood information and estimation of a physiological condition have been enabled in recent years as described above, it is required to take a given motion to estimate a physiological condition and this may put much strain on the user.

The disclosure thus proposes an information processing device, an information processing method and a program that are novel and improved and that makes it possible to easily estimate a physiological condition of a user in motions in daily life.

Solution to Problem

According to the present disclosure, an information processing device is provided. The information processing device includes: a physiological condition estimator configured to estimate a physiological condition of a user who is in a given physical state based on a first blood flow amount change of the user and a second blood flow amount change of the user or another user in the given physical state, the first blood flow amount change and the second blood flow amount change are acquired by a blood flow information detector.

Also, according to the present disclosure, an information processing method is provided. The information processing method includes: estimating a physiological condition of a user who is in a given physical state based on a first blood flow amount change of the user and a second blood flow amount change of the user or another user in the given physical state, the first blood flow amount change and the second blood flow amount change are acquired by a blood flow information detector.

Moreover, according to the present disclosure,
an program is provided. The program causes a computer to implement a function of estimating a physiological condition of a user who is in a given physical state based on a first blood flow amount change of the user and a second blood flow amount change of the user or another user in the given physical state, the first blood flow amount change and the second blood flow amount change are acquired by a blood flow information detector.

Brief Description of Drawings

FIG. 1 is a diagram illustrating an example of a mode of an information processing device 10 according to a first embodiment of the disclosure.
FIG. 2 is a block diagram illustrating a functional configuration of the information processing device 10 according to the same embodiment.

FIG. 3 is a block diagram illustrating a functional configuration of a controller 100 according to the same embodiment.

FIG. 4 is a block diagram illustrating a functional configuration of a blood flow sensor 300 according to the same embodiment.

FIG. 5 is an explanatory view for explaining a blood flow measurement method that is applied to the same embodiment.

FIG. 6 is an explanatory view for explaining a method of estimating biological information according to the same embodiment.

FIG. 7 is a flowchart illustrating an example of an information processing method according to the same embodiment.

FIG. 8 is an explanatory view illustrating an example of output of an estimation result according to the same embodiment.

FIG. 9 is an explanatory view illustrating another example of the output of an estimation result according to the same embodiment.

FIG. 10 is an explanatory view illustrating still another example of the output of an estimation result according to the same embodiment.

FIG. 11 is a block diagram illustrating a functional configuration of an information processing device 10a according to a second embodiment of the disclosure.

FIG. 12 is an explanatory view for explaining a blood flow measurement method that is applied to the same embodiment.

FIG. 13 is an explanatory view for explaining another example of the blood flow measurement method that is applied to the same embodiment.

FIG. 14 is a block diagram illustrating a functional configuration of a controller 100a according to the same embodiment.

FIG. 15 is a flowchart illustrating an example of an information processing method according to the same embodiment.

FIG. 16 is a block diagram illustrating a functional configuration of an information processing device 10a according to a modification of the second embodiment of the disclosure.

FIG. 17 is an explanatory view for explaining the modification of the second embodiment of the disclosure.

FIG. 18 is an explanatory view (1) for explaining an example of machine learning according to the same embodiment of the disclosure.

FIG. 19 is an explanatory view (2) for explaining an example of machine learning according to the same embodiment.

FIG. 20 is a block diagram illustrating a functional configuration of a controller 100b according to a fourth embodiment of the disclosure.

FIG. 21 is a flowchart illustrating an example of an information processing method according to the same embodiment.

FIG. 22 is an explanatory view illustrating an example of output of an estimation result according to the same embodiment.

FIG. 23 is a block diagram illustrating a functional configuration of a controller 100c according to a fifth embodiment of the disclosure.

FIG. 24 is a flowchart illustrating an example of an information processing method according to the fifth embodiment.

FIG. 25 is an explanatory view illustrating an example of output of an estimation result according to the same embodiment.

FIG. 26 is an explanatory view for explaining a sixth embodiment of the disclosure.

FIG. 27 is an explanatory view illustrating an example of a hardware configuration of an information processing device900 according to an embodiment of the disclosure.

Description of Embodiments

With reference to the accompanying drawings, preferred embodiments of the disclosure will be described in detail below. Components having substantially the same functions herein and the drawings may be denoted with the same reference signs and thus redundant description will be omitted.

In the specification and drawings, multiple components having substantially the same or similar functional configurations may be distinguished from one another by adding different numbers to the end of the same signs. Note that, when it is not particularly necessary to distinguish multiple components having substantially the same or similar functional configurations, only the same numbers are added. Similar components among different embodiments may be distinguished from one another by adding different alphabet letters to the end of the same signs. When it is not particularly necessary to distinguish similar components, only the same reference signs are added.

Description will be given in the following order.

1. Background of Creation of Embodiments of Disclosure
2. First Embodiment
3. Second Embodiment

4. Third Embodiment

5. Fourth Embodiment

6. Fifth Embodiment

7. Sixth Embodiment

8. Summary

9. About Hardware Configuration

10. Supplementary

<<1. Background of Creation of Embodiments of Disclosure>>

First of all, before detailed description of the embodiments of the disclosure is given, the background of creation of embodiments of the disclosure by the inventors will be described.

As described above, in recent years, there has been a progress in development of techniques enabling casual measurement of an amount of blood flow, a rate of blood flow, etc., that are blood flow information blood flow of a user. Based on an amount of blood flow that is measured by such a technique, it is possible to determine a physiological condition (such as dehydration or anemia) of a user or diagnose a disease. Suppose herein that determination of a physiological condition includes diagnosing a disease from which the user suffers and predicting a risk of epidemic.

For example, the technique disclosed in Patent Literature 1 above includes inducing the user to take a given motion, that is, lifting an arm from a lowered state and then keeping the arm in the lifted state, detecting change in the amount of blood flow at that time and thereby determining whether the user has dehydration. In the technique, whether the user takes the given motion is detected by an acceleration sensor that is worn on part of the body of the user.

According to the technique of Patent Literature 1, because it is required to cause the user to execute the given motion, the above-described motion is required each time a physiological condition of the user is estimated. This results in putting much strain on the user.

It may be difficult for the user to be conscious of the symptom of dehydration, or the like. Thus, as in Patent Literature 1 above, even when the user takes the given motion only after the user is conscious of the symptom and a physiological condition is estimated, the symptom of the user may progress and it may be too late. Particularly for elderly people and users with cardiac diseases, etc., knowing that they have dehydration as soon as possible in daily life leads to protection their lives and therefore it is required to easily estimate a physiological condition in motions in daily life (daily motions). Furthermore, as described above, taking the given motion consciously may be much strain on such users.

Thus, while continuing sincere consideration under such circumstances, the inventors reached creation of an information processing device according to an embodiment of the disclosure that enables easy estimation of a physiological condition of a user in daily motions. Details of the embodiment of the disclosure created by the inventors will be described below.

<<2. First Embodiment>>

<2.1 Mode of Information Processing Device 10>

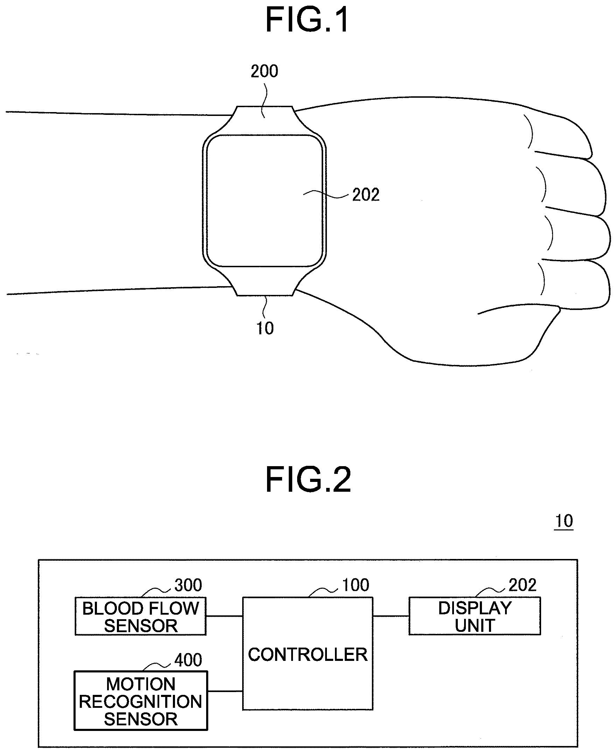

First of all, a mode of an information processing device 10 according to a first embodiment of the disclosure will be described with reference to FIG. 1. FIG. 1 is a diagram illustrating the example of the information processing device 10 according to the present embodiment.

As illustrated in FIG. 1, the information processing device 10 according to the present embodiment is realized, for example, using a wristwatch-type wearable device that is wearable around a wrist of the user. Specifically, the information processing device 10 includes a band unit 200 having a belt-like shape and a display unit (output unit) 202 that is arranged on part of the outer circumference of the band unit 200. Furthermore, although illustration is omitted, a blood flow sensor 300 (blood flow information detector) (refer to FIG. 2) that detects blood flow information on the user (here, including the user wearing the information processing device 10 and another user wearing the information processing device 10) may be arranged on part of the inner circumference of the band unit 200 and a controller 100 (refer to FIG. 2) that controls operations of the information processing device 10 may be arranged in the band unit 200. Note that details of these function units of the information processing device 10 will be described below.

Furthermore, although illustration is omitted from FIG. 1, a motion recognition sensor 400 (refer to FIG. 2) consisting of various sensors (for example, motion sensors (such as a three-axis acceleration sensor, a three-axis gyro sensor, and a geomagnetic sensor), a barometric pressure sensor, a temperature sensor, a myoelectric sensor, and a positioning sensor (such as a GPS (Global Positioning System)) is arranged in the information processing device 10. In addition, although illustration is omitted from FIG. 1, for example, an output device (output unit), such as a speaker, an oscillation module or a light emitter, may be arranged in the information processing device 10.

For example, the information processing device 10 may be a device that is wearable on part of the body of the user or another user or an implant device (implant terminal device) that is inserted into the body of the user or another user. Specifically, the information processing device 10 is not limited to wrist-watch type wearable devices like that described above and various types of wearable devices, such as a HMD (head mounted display) type, an eye-wear type, an ear-device type, an anklet type, a collar type, a pad type, a badge type and a cloth type, are usable. The information processing device 10 may be a portable device, such as a smartphone or a tablet PC (Personal Computer).

<2.2 Functional Configuration of Information Processing Device 10>

The example of the mode of the information processing device 10 according to the present embodiment has been described. With reference to FIG. 2, an example of a functional configuration of the information processing device 10 according to the present embodiment will be described. FIG. 2 is a block diagram illustrating a functional configuration of the information processing device 10 according to the present embodiment. As illustrated in FIG. 2, the information processing device 10 according to the present embodiment mainly includes, for example, the controller 100, the display unit 202, a blood flow sensor 300, and the motion recognition sensor 400. Details of each functional unit of the information processing device 10 according to the present embodiment will be described below.

(Controller 100)

The controller 100 is, for example, arranged in the above-described band unit 200 and is capable of controlling each functional unit of the information processing device 10. The controller 100 is realized, for example, using hardware, such as a CPU (Central Processing Unit), a ROM (Read Only Memory), or a RAM (Random Access Memory). Part of the function of the controller 100 may be provide by an external server (illustration omitted), or the like. The detailed configuration of the controller 100 will be described below.

(Display Unit 202)

The display unit 202 is arranged, for example, in part of the outer circumference of the band unit 200 described above, is controlled by the controller 100 described above, and thus is capable of displaying (outputting) an estimated physiological condition (inference result) of the user (here, including the user wearing the information processing device 10 and another user wearing the information processing device 10) to the user, etc. The display unit 202 can be realized from, for example, a display device, such as a liquid crystal display (LCD) device or an OELD (Organic Light Emitting Diode) device.

(Blood Flow Sensor 300)

The blood flow sensor 300 is, for example, arranged in part of the inner circumference of the band unit 200 described above and is capable of acquiring blood flow information on the user (here, including the user wearing the information processing device 10 and another user wearing the information processing device 10). Note that the detailed configuration of the blood flow sensor 300 will be described below.

(Motion Recognition Sensor 400)

The motion recognition sensor 400 is arranged on the inner side of the band unit 200 described above and is capable of acquiring information on motions of the user (here, including the user wearing the information processing device 10 and another user wearing the information processing device 10). Specifically, the motion recognition sensor 400 may include a motion sensor (illustration omitted) for detecting motions of the user. The motion sensor is capable of recognizing a motion of the user by acquiring sensing data indicating change in the acceleration, posture, etc., that occurs in association with the motion of the user. More specifically, the motion sensor can be realized using an acceleration sensor, a gyro sensor, a geomagnetic sensor (illustration omitted), etc.

The motion recognition sensor 400 may contain a positioning sensor (illustration omitted) together with the motion sensor described above. The positioning sensor is a sensor that detects a position of the user, and, specifically, a GNSS (Global Navigation Satellite System) receiver, or the like, is usable. In this case, the positioning sensor is able to generate sensing data indicating the longitude and latitude of the current position of the user (here, including the user wearing the information processing device 10 and another user wearing the information processing device 10) based on signals from GNSS satellites. In the present embodiment, for example, because it is possible to detect a relative positional relationship of the user from RFID (Radio Frequency Identification), a Wi-Fi access point, information on a radio base station, etc., it is possible to use such a communication device as the aforementioned positioning sensor.

In the motion recognition sensor 400, a barometric pressure sensor that detects change in the barometric pressure, a temperature sensor and a pressure sensor that detect a temperature and a humidity of the surrounding environments of the user (here, including the user wearing the information processing device 10 and another user wearing the information processing device 10), etc., may be arranged. In the present embodiment, in the information processing device 10, other various biological information sensors (illustration omitted) other than the blood flow sensor 300 may be arranged. For example, the various biological information sensors may include one or more sensors that measure brain waves, breathing, a myopotential, a skin temperature, sweating, blood pressure, blood oxygen level, etc.

In the above description, the controller 100, the display unit 202, the blood flow sensor 300, and the motion recognition sensor 400 are described as ones included by the information processing device 10; however, the present embodiment is not limited to this. In the present embodiment, for example, one or more of the above-described function units and the remaining function units may be configured as different units and may be connected with each other by radio communication, or the like.

<2.3 Functional Configuration of Controller 100>

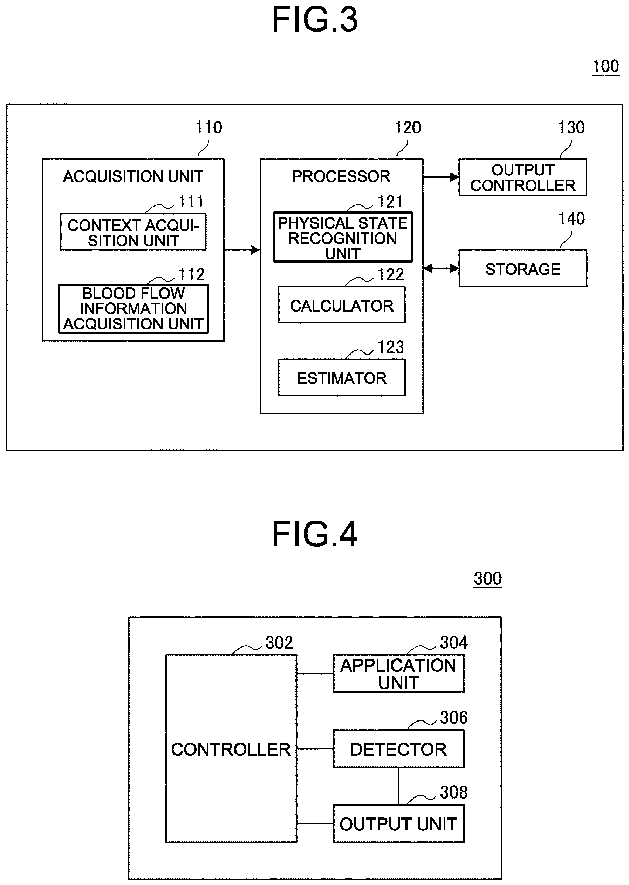

The example of the functional configuration of the information processing device 10 according to the present embodiment has been described. Subsequently, with reference to FIG. 3, an example of a functional configuration of the controller 100 of the information processing device 10 according to the present embodiment will be described. FIG. 3 is a block diagram illustrating a functional configuration of the controller 100 of the information processing device 10 according to the present embodiment. As illustrated in FIG. 3, the controller 100 of the information processing device 10 according to the embodiment mainly includes, for example, an acquisition unit 110, a processor 120, an output controller 130, and a storage 140. Details of each function unit of the controller 100 will be described below.

(Acquisition Unit 110)

The acquisition unit 110 has a function of acquiring various types of information that are used in a process that is performed by the processor 120 and outputting the acquired various types of information to the processor 120. Specifically, as illustrated in FIG. 3, the acquisition unit 110 includes a context acquisition unit 111 and a blood flow information acquisition unit 112. Details of the context acquisition unit 111 and the blood flow information acquisition unit 112 will be described below.

~Context Acquisition Unit 111~

The context acquisition unit 111 has a function of acquiring context information on the user (here, including the user wearing the information processing device 10 and another user wearing the information processing device 10) and outputting the context information to the processor 120. The context information here refers to information on activity of the user or environment around the user. For example, the context information can contain the posture of the user (for example, an upright positon, a seated position, or the like), a motion of the user (for example, walking, an exercise, households, eating, drinking, studying, talking, or driving) and the history of behaviors of the user (for example, transportation, time, or schedule). Furthermore, the context information may contain profile information on the user (such as, for example, the gender, the age, an exercise experience history, and a case history). The context information may contain information on the category of environments around the user (indoor and outdoor), a region, a season, a temperature, a humidity, etc.

The context acquisition unit 111 is capable of acquiring such information as that described above, for example, from the motion recognition sensor 400 described above. The context acquisition unit 111 may acquire such information as that described above via a direct input by the user or wireless communication from the external server (illustration omitted) that stores profile information on the user, etc., via radio communication, or the like.

~Blood Flow Information Acquisition unit 112~

The blood flow information acquisition unit 112 has a function of acquiring blood flow information on the blood flow of the user (here, including the user wearing the information processing device 10 and another user wearing the information processing device 10) from the blood flow sensor 300 and outputting the blood flow information to the processor 120. In the present embodiment, for example, an amount of blood flow can be taken as blood flow information on the user. Here, the amount of blood flow refers to an amount of blood passing through one or more blood vessels per unit of time in a measurement area (part of the body) of the user. In the present embodiment, change in the amount of blood flow of another user over time, etc., is stored in the storage 140 to be described below as information to be used to estimate a physiological condition of the user.

(Processor 120)

The processor 120 has a function of processing various type of information that are acquired by the acquisition unit 110 and outputting the information to the output controller 130 to be described below. Specifically, as illustrated in FIG. 3, the processor 120 includes a physical state recognition unit (state detector) 121, a calculator 122, and an estimator (physiological condition estimator) 123. Details of each function unit of the processor 120 will be described below.

~Physical State Recognition Unit 121~

The physical state recognition unit 121 has a function of recognizing that the user enters a given physical state based on the context information and the blood flow information of the user (including the user wearing the information processing device 10 and another user wearing the information processing device 10 here). The given physical state is, for example, a state in which blood vessels of the user are pressurized or contracted and the amount of blood flow decreases (blood flow amount decreased state below) and taking a given motion leads to the blood flow amount decreased state. Specifically, for example, a motion of lifting an arm or a motion of squeezing the arm in daily life that causes muscles to pressurize blood vessels is taken as a motion leading to the blood flow amount decreased state. Accordingly, in the present embodiment, for example, detecting the motion of lifting an arm (the given motion) taken by the user based on the sensing data obtained by the various sensors makes it possible to recognize that the user is in the blood flow amount decreased state.

Furthermore, the physical state recognition unit 121 may recognize that the user is in the blood flow amount decreased state based on the context information described above.

More specifically, hanging on to a strap on a train can be taken as the motion of lifting an arm that is taken by the user. Such a motion, for example, is detectable from the speed of travel by train or vibrations of the arm based on the sensing data of the acceleration sensor. For example, a motion of hanging laundry that is taken by the user can be taken as the motion of lifting an arm by the user. Such a motion is detectable by, for example, recognizing that the user is at the balcony of the place of the user based on the sensing data of the positioning sensor and recognizing that an arm is lifted based on sensing data of the barometric pressure sensor. Furthermore, as another example, for example, a motion of carrying a heavy bag at a supermarket can be taken as the motion of squeezing the arm of the user. Such a motion is detectable, for example, according to contraction of muscle fibers of the user based on sensing data of the myoelectric sensor, vibrations of the arm based on sensing data of the acceleration sensor, etc., and by recognizing that the user is in a supermarket based on sensing data of the positioning sensor. For example, a training motion in a gym can be taken as the motion of squeezing the arm of the user. Such a motion is detectable, for example, by recognizing that the user is in a gym based on sensing data of the positioning sensor and recognizing that the arm is lifted based on sensing data of the barometric pressure sensor.

In other words, in the present embodiment, because the physical state recognition unit 121 detects a motion of the user that leads to the blood flow amount decreased state in daily life, it is possible to easily estimate a physiological condition of the user without requiring the user to execute the given motion. Thus, in the present embodiment, because the user is not required to execute the given motion, it is possible to avoid putting much strain on the user.

~Calculator 122~

When it can be recognized that the user (here, including the user wearing the information processing device 10 and another user wearing the information processing device 10) is in the blood flow amount decreased state, the calculator 122 calculates an index (blood flow volume recovery index) on recovery of the amount of blood flow from change in the amount of blood flow over time based on the blood flow information from the blood flow information acquisition unit 112. Note that the calculated index may be stored in the storage 140 to be described below. Furthermore, the calculator 122 outputs the aforementioned calculated index to the estimator 123 to be described below. Details of the index on the blood flow amount recovery index that is calculated by the calculator 122 will be described below.

~Estimator 123~

The estimator 123 estimates a physiological condition of the user based on the above-described blood flow volume recovery index that is calculated by the calculator 122 and outputs an estimation result to the output controller 130 to be described below. The estimator 123 may estimate a physiological condition of the user with reference to a database that is obtained by machine learning and furthermore and estimate a physiological condition of the user based on the context information described above. Details of an estimation method performed by the estimator 123 will be described below.

(Output Controller 130)

The output controller 130 has a function of presenting the aforementioned estimation result to the user, etc. The output controller 130, for example, controls an output device, such as the display unit 202, and presents the estimation result in a form of sound, vibrations, an image, blinks, or the like. In the present embodiment, the output device can be, for example, the display unit 202, a speaker, a vibration module, a light emitting device (illustration omitted), or the like, that is arranged in the information processing device 10.

(Storage 140)

The storage 140 stores programs and information, etc., for executing various types of processing by the controller 100 and information obtained by the processing. The storage 140 is realized using, for example, a nonvolatile memory, such as a flash memory.

<2.4 Functional Configuration of Blood Flow Sensor 300>

The example of the functional configuration of the controller 100 according to the present embodiment has been described. With reference to FIG. 4, an example of a functional configuration of the blood flow sensor 300 according to the present embodiment will be described. FIG. 4 is a block diagram illustrating the functional configuration of the blood flow sensor 300 according to the present embodiment. As illustrated in FIG. 4, the blood flow sensor 300 mainly includes, for example, a controller 302, an application unit 304, a detector 306, and an output unit 308. Details of each function unit of the blood flow sensor 300 will be described below.

(Controller 302)

The controller 302 controls application of light by the application unit 304 to be described below, controls reading (sampling) by the detector 306 to be described below, etc., that is, controls general measurement performed by the blood flow sensor 300. Furthermore, the controller 302 may incorporate a clock system (illustration omitted) for knowing accurate time in order to output sensing data in association with time to the controller 100.

(Application unit 304)

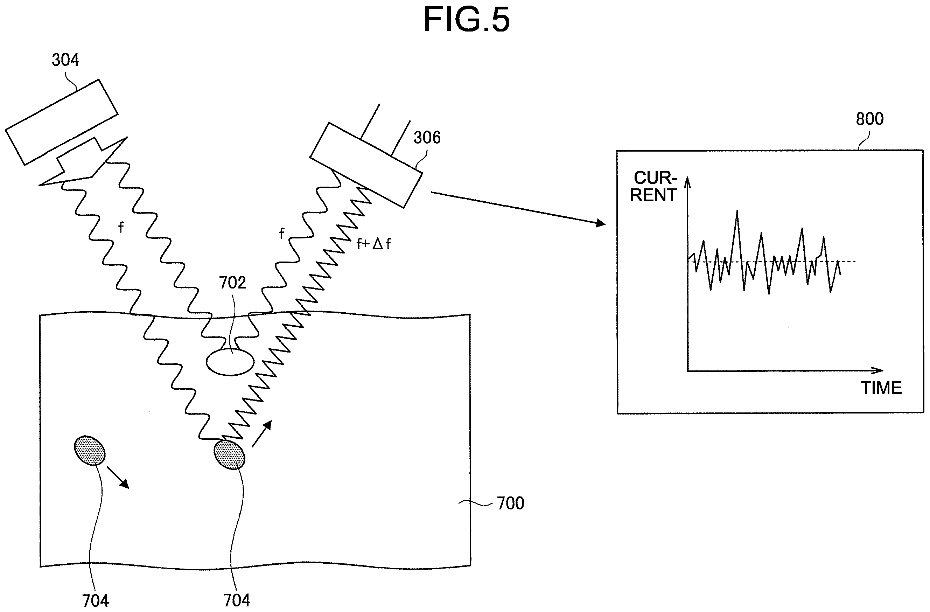

The application unit 304 applies an application light having a given wavelength to a measurement area (part of the body) 700 of the user (here, including the user wearing the information processing device 10 and another user wearing the information processing device 10) (refer to FIG. 5). It is possible to appropriately select a wavelength of the application light that is applied by the application unit 304 and applies, for example, light having a wavelength of approximately 800 nm to 900 nm. For example, a laser device, or the like, is usable as the application unit 304 to apply coherent light. The above-described controller 302 makes it possible to control timing when the application unit 304 applies application light, duration of application, interval of application, intensity, etc.

(Detector 306)

The detector 306 detects light that is reflected from the measurement area 700 of the user (here, including the user wearing the information processing device 10 and another user wearing the information processing device 10). The detector 306 includes, for example, a photodiode (photodetector (PD)) and the detector 306 converts the intensity of received light into an electric signal (sensing data) and outputs the electric signal to the output unit 308 to be described below. A CCD (Charge Coupled Devices) sensor, a CMOS (Complementary Metal Oxide Semiconductor) sensor, or the like, is usable as the detector 306.

(Output Unit 308)

The output unit 308 has a function of outputting the sensing data obtained by the detector 306 to the controller 100 described above.

Furthermore, the method of measuring blood flow information performed by the blood flow sensor 300 described above will be described with reference to FIG. 5. FIG. 5 is an explanatory view for explaining the blood flow measurement method that is applied to the present embodiment. A laser Doppler blood flow measurement technique and a technique of analyzing a velocity distribution using dynamic light scattering (DLS) are taken as examples of the blood flow measuring method according to the present embodiment.

The method of measuring blood flow information according to the present embodiment is, for example, a method in which, when light is applied from the application unit 304 to a measurement area (part of the body) 700 of the user (here, including the user wearing the information processing device 10 and another user wearing the information processing device 10), because the frequency of the light scattered by a scattering substance (mainly red blood cells) moving through blood vessels of the user shifts due to the Doppler effect and the scattered light interferes with the light scattered by a still part, velocity information on the scattering substance is obtained from a beat frequency of the interference light. In the present embodiment, for example, the detector 306 receives the interference light and calculates blood flow information from the distribution of Doppler shift frequencies of the received interference light.

Specifically, as illustrated in FIG. 5, light with a frequency f that is applied by the application unit 304 to the measurement area 700 of the user is scattered by still tissue 702 that is still, such as the skin or subcutaneous tissue of the user, the scattered light maintains the frequency f. On the other hand, the light with the frequency f that is applied to the measurement area 700 of the user is scattered by a scattering substance moving through blood vessels of the user (for example, red blood cells are taken and blood cells are a substance having a diameter of 8 $\mu$m to 10 $\mu$m) 704, the scattered light has a frequency shift because of the positional shift of the scattering substance 704 and the Doppler effect and has a frequency f+$\Delta$f. The scattered light with the frequency f that is scattered by the still tissue 702 and the scattered light with the frequency f+$\Delta$f that is scattered by the traveling scattering substance 704 interfere with each other and accordingly the detector 306 is able to detect the interference light with a light beat. Note that, in general, the shift frequency $\Delta$f is significantly smaller than the frequency f of the applied light. By processing the interference light (detection signal) that is detected by the detector 306, it is possible to obtain blood flow information.

By processing the interference light (detection signal) that is detected by the detector 306 like that denoted by a sign 800 in FIG. 5, it is possible to obtain blood flow information. Note that, because the detection signal 800 is a signal of superimposed light beats of multiple different frequencies due to the scattered light from the scattering substance 704 in blood vessels with multiple different motions, the detection signal 800 looks like an irregular signal like white noise as illustrated in FIG. 5. The detection signal 800 is, however, a signal of superimposed interference beats of multiple frequencies as described above, and therefore performing frequency analytical processing on the detection signal makes it possible to acquire velocity distribution information on particle motions that cause a frequency shift, that is, a Doppler shift. Thus, in the laser Doppler blood flow measurement method, by knowing the velocity distribution of the scattering substance 704, such as red blood cells in the blood flow, it is possible to acquire blood flow information, such as an amount of blood flow.

Alternatively, when coherent light is emitted to the measurement area 700 of the user, because of interference of the light scattered by the scattering substance in the measurement area 700, it is possible to obtain a pattern referred to as a speckle pattern. A change occurs in the speckle pattern due to scattering by the traveling scattering substance 704. Thus, in the present embodiment, the blood flow sensor 300 may be a sensor that acquires such a speckle pattern. Specifically, for example, it is possible to acquire blood flow information by calculating velocity information on the scattering substance 704, such as red blood cells in the blood flow, based on information on fluctuations in the speckle pattern of an ear lobe, a fingertip, or the like. In this case, the blood flow sensor 300 is not limited to being incorporated in the information processing device 10 in a mode of a wristwatch-type wearable device like that illustrated in FIG. 1, and the blood flow sensor 300 may be incorporated in the information processing device 10 in a mode of an imaging device capable of acquiring the above-described speckle pattern.

Photoplethysmography (PPG) can be taken as another example of the blood flow measurement method according to the present embodiment. Specifically, PPG is a measurement method utilizing change in the amount of absorption of light by a blood vessel in association with a change in the volume of the blood vessel (vessel) that occurs in association with pumping of blood by the heart. Specifically, when the application unit 304 applies light to the measurement area of the user, because the applied light is selectively absorbed mainly into red blood cells in blood of the user, the volume of absorption of light is proportional to the amount of blood (specifically, amount of blood of tissue). Thus, in PPG, detecting light reflected or transmitted via the skin, blood vessels, of the user, etc., by the detector 306 makes it possible to obtain a change in the amount of blood passing per pulse from the result of detection and acquire an amount of blood flow per unit of time. The method of measuring a blood flow velocity that is applicable to the embodiment of the disclosure is not limited to these measurement methods and another measurement method may be used.

<2.5 Method of Estimating Physiological Condition>

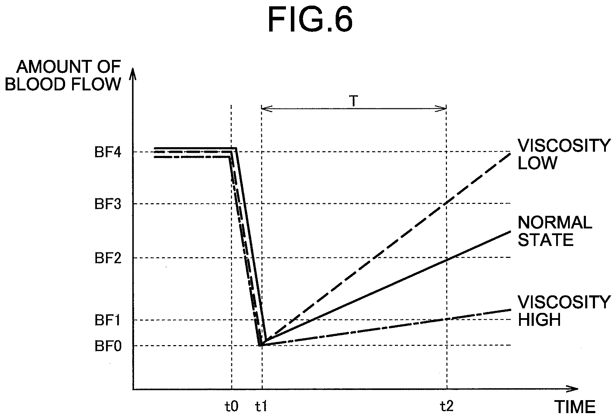

The example of the functional configuration of the blood flow sensor 300 according to the present embodiment has been described. With reference to FIG. 6, an overview of the method of estimating a physiological condition of a user according to the present embodiment will be described. FIG. 6 is an explanatory view for explaining a method of estimating biological information according to the present embodiment and, specifically, a graph indicating change in the amount of blood flow at and after the blood flow amount decreased state.

When the user takes a motion, such as lifting an arm, and thus the blood vessel is contracted, the amount of blood flow decreases temporarily but the heart of the user functions to increase the amount of blood flow in order to restore the amount of blood flow to a steady state (homeostasis). For example, when the user lifts an arm at a time t0 presented in FIG. 6, the amount of blood flow decreases from a steady value (normal value) BF4 because of the aforementioned motion from time t0 to time t1. When the amount of blood flow decreases as described above, however, the amount of blood flow increases at and after time t1 (blood flow amount recovery period) in the heart and is recovered approximately to the aforementioned steady value BF 4.

As illustrated in FIG. 6, in the blood flow amount recovery period, when the blood viscosity is high, because flowing is not easy compared to the case where the blood viscosity is in the steady state (normal state), a recovery time taken to recover the amount of blood flow approximately to the constant value (normal value) BF4 gets longer. On the other hand, when the blood viscosity is low, because flowing is easy compared to the case where the blood viscosity is in the constant state (normal state), a time taken to recover the amount of blood flow approximately to the constant value BF4 gets shorter.

When the physiological condition of the user is dehydration, water in blood decreases and the blood viscosity increases (the blood viscosity increases because blood plasma with a low viscosity decreases). Accordingly, in such a case, there is a tendency that the aforementioned recovery time gets longer compared to the case where the blood viscosity is in the constant state (normal state). On the other hand, when the physiological condition of the user is anemia, the number of red blood cells decreases and the blood viscosity decrease. Accordingly, in such a case, there is a tendency that the aforementioned time of recovery gets shorter than that in the case where the blood viscosity is in the constant state. Thus, in the present embodiment, it is possible to acquire information on the aforementioned recovery time as the blood flow amount recovery index and estimate a physiological condition of the user based on the information on the recovery time.

Specifically, in the present embodiment, a physiological condition of the user is estimated based on a blood flow amount recovery slope (blood flow recovery index) (first blood flow amount change) that is a slant of the amount of blood flow with respect to the time in the blood flow amount recovery period, which is the slope obtained after the amount of blood flow turns to an increase after decreasing temporarily because of the blood flow amount decreased state. More specifically, the blood flow amount recovery slope is a slant that is calculated by dividing the sum of amounts of blood flow until elapse of a given time T (for example, a time T from a time t1 to a time t2 illustrated in FIG. 6) in the blood flow recovery period by the time T. In the present embodiment, the given time T is appropriately selectable.

More specifically, when the blood viscosity is in the constant state (normal state), a blood amount recovery slope (second blood flow amount recovery slope) $\Delta B1$ is representable by Equation (1) below. Note that, in the present embodiment, the blood flow amount recovery slope $\Delta B1$ may be a blood flow amount slope of the subject user at the time when the blood flow viscosity is in the constant state, an average of multiple blood flow amount slopes of the subject user, or a blood flow amount slope of another or other users other than the user at the time when the blood flow viscosity is in the constant state or an average of blood flow amount slopes of another or other users. Note that these values are acquired previously.

$$\Delta B1 = \frac{BF2 - BF0}{T} \tag{1}$$

When the blood viscosity is low, a blood recovery slope (first blood flow amount recovery slope) $\Delta B2$ is representable by, for example, Equation (2) below. Note that the blood flow amount recovery slope $\Delta B2$ can be a blood flow amount slope of the subject user or an average of multiple blood flow amount slopes of the subject user.

$$\Delta B2 = \frac{BF3 - BF0}{T} \tag{2}$$

When the blood viscosity is high, a blood amount recovery slope $\Delta B3$ is representable by Equation (3) below. Note that the blood flow amount recovery slope $\Delta B3$ can be a blood flow amount slope of the subject user or an average of multiple blood flow amount slopes.

$$\Delta B3 = \frac{BF1 - BF0}{T} \tag{3}$$

Thus, in the present embodiment, when a blood flow amount recovery slope is larger than the blood flow amount recovery slope ΔB1 (a second blood flow amount change) (reference value) (for example, in the case of ΔB2), it is possible to estimate that the user has anemia in which the blood viscosity is low.

On the other hand, in the present embodiment, when the blood flow amount recovery slope is smaller than the blood flow amount recovery slope ΔB1 (for example, in the case of ΔB3), it is possible to estimate that the user has dehydration in which the blood viscosity is high.

Note that, the present embodiment is not limited to estimating a physiological condition of the user by comparing the blood flow amount recovery slope ΔB1 to the blood flow amount recovery slope ΔB1 as described above. For example, a physiological condition of the user may be estimated by comparing the blood flow amount recovery slope ΔB to a given value range that is defined previously based on the blood flow amount recovery slope ΔB1 (for example, a value range in which the blood flow amount recovery slope ΔB1 serves as a median) (reference value).

In the present embodiment, the blood flow amount recovery slope ΔB1 in the case where the blood viscosity is in the steady state (normal state) (referred to as a steady (normal) blood flow amount recovery slope in the following description) can be obtained, for example, based on change in the amount of blood flow over the blood flow amount recovery period (steady (normal) change over time) obtained when a given time elapses after the user or another user drinks or eats. The steady blood flow amount recovery slope may be acquired based on an average of multiple changes in the amount of blood over time that are acquired in a given period (a day, a week, or a month), or the like, or may be acquired based on an average of multiple changes in the amount of blood flow of multiple other users (for example, multiple user with the same profile as that of the user, such as males over 20) over time. Note that details of a method of acquiring a constant state blood flow amount recovery slope, that is, a method of recognizing the case where the blood viscosity is constant (normal) will be described below.

In the present embodiment, it is possible to increase accuracy in estimating a physiological condition by referring to profile information on the user (such as the gender, the age, and a case history (such as economy syndrome)), information on environments around the user (such as the temperature and humidity), positional information (outdoor or indoor), etc., that are the above-described context information.

The present embodiment is not limited to using a blood flow amount recovery slope like the one described as a blood flow amount recovery index, and a physiological condition of the user may be estimated using another index. For example, a physiological condition of the user may be estimated using, as an index, a shape of a peak appearing in change in the amount of blood flow of the user (waveform), an integral of blood flow amounts in a given time, or the like. Furthermore, in the present embodiment, instead of calculating a blood flow amount recovery slope, a database obtained by performing machine learning on multiple changes in the amount of blood flow of the user or another user that were acquired in the past and that are labelled (labelled with profile information, physiological conditions, or the like) may be referred to. In this case, for example, it is possible to estimate a physiological condition of the user based on a feature value that is extracted from change in the amount of blood flow over time that belongs to the same cluster as that of the user and a newly-acquired feature value that is extracted from change in the amount of blood flow of the user over time.

<2.6 Information Processing Method>

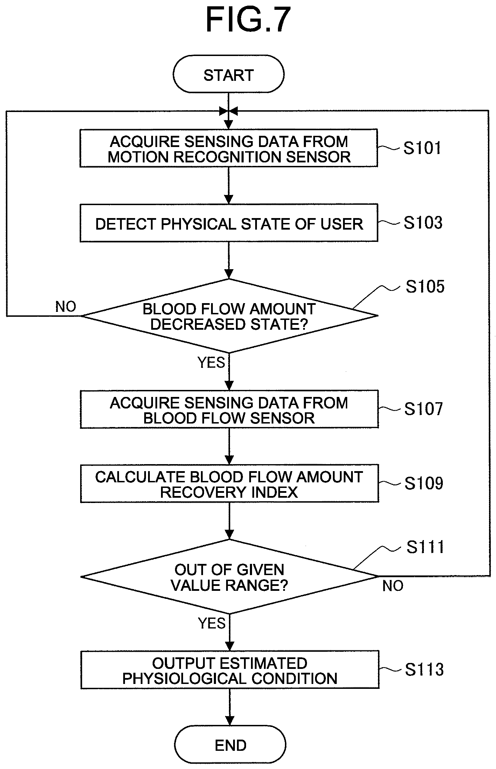

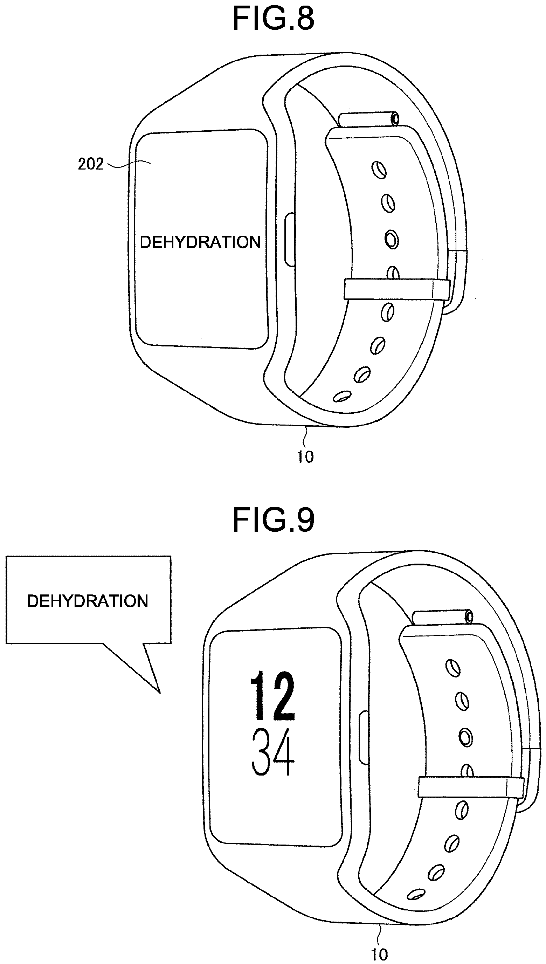

With reference to FIGS. 7 to 10, an information processing method according to the present embodiment will be described. FIG. 7 is a diagram illustrating a flowchart of the information processing method according to the present embodiment. FIGS. 8 to 10 are explanatory views illustrating examples of an output of an estimation result according to the present embodiment.

The information processing device 10 according to the present embodiment is worn on part of the body of the user who lives a daily life and performs information processing below in order to estimate a physiological condition of the user in daily life of the user. In other words, in the present embodiment, the aforementioned estimation of a physiological condition is not performed in a special situation or the user does not intentionally take a special motion for the estimation.

First of all, as illustrated in FIG. 7, the information processing method according to the present embodiment incudes multiple steps from step S101 to step S113. Each step of the information processing method according to the present embodiment will be described below.

(Step S101)

First of all, the controller 100 acquires sensing data from the motion recognition sensor 400.

(Step S103)

Based on the sensing data that is acquired from the motion recognition sensor 400 at step S101 described above, the controller 100 detects a physical state of the user. More specifically, based on the sensing data, the controller 100 detects a motion of the user in daily life, such as lifting an arm or immobilizing an arm.

(Step S105)

The controller 100 determines whether the physical state (specifically, the motion of the user) that is detected at step S103 described above corresponds to the blood flow amount decreased state. When it is determined that the physical state corresponds to the blood flow amount decreased state, the controller 100 proceeds the process to step S107 to be described below and, when it is determined that the physical state does not correspond to the blood flow amount decreased state, the controller 100 returns to step S101 described above. Specifically, in the present embodiment, the controller 100 is able to make the above-described determination based on whether the motion of the user that is detected at step S103 described above corresponds to a motion that leads to a predefined state where blood vessels of the user are pressurized or contracted.

(Step S107)

The controller 100 acquires sensing data (change in the amount of blood flow over time) from the blood flow sensor 300.

(Step S109)

Based on the sensing data that is acquired from the blood flow sensor 300 at step S107 described above, the controller 100 calculates a blood flow amount recovery index, that is, a blood flow amount recovery slope.

(Step S111)

The controller 100 further determines whether the blood flow amount recovery index (blood flow amount recovery slope) that is calculated at step S109 described above is out of the given value range. When it is determined that the blood flow amount recovery index is out of the given value range, the controller 100 proceeds the process to step S113 described above and, when it is determined that the blood flow amount recovery index is not out of the given value range, the controller 100 returns to step S101.
For example, it is possible to set, for the given value range, a value range in which the steady (normal) blood flow amount recovery slope is a median. At step S111, instead of determining whether the blood flow amount recovery slope that is calculated at step S109 is out of the given value range, the controller 100 may make determination using a ratio indicating how much the blood flow amount recovery slope that is calculated at step S109 deviates from the steady blood flow amount recovery slope, or the like.

(Step S113)

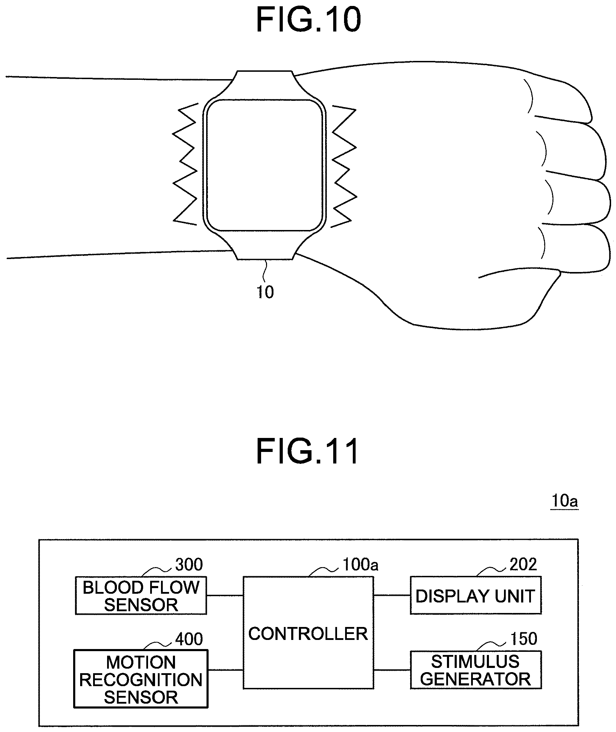

When the blood flow amount recovery index (blood flow amount recovery slope) that is calculated at step S109 is larger than an upper limit value of the given value range at step S111, the controller 100 estimates that the physiological condition of the user is anemia. On the other hand, when the blood flow amount recovery index (blood flow amount recovery slope) that is calculated at step S109 is larger than a lower limit value of the given value range at step S111, the controller 100 estimates that the physiological condition of the user is anemia. The controller 100 outputs the estimated physiological condition (estimation result) to the user and ends the process.
When it is estimated that the physiological condition of the user is dehydration at step S113, for example, as illustrated in FIG. 8, the information processing device 10 may output the estimation result to the user by displaying a word "Dehydration". As illustrated in FIG. 9, for example, the information processing device 10 may output the estimation result to the user by outputting voice "Dehydration" via a speaker (illustration omitted). When it is estimated that the physiological condition of the user is dehydration or anemia at step S113 described above, for example, as illustrated in FIG. 10, the information processing device 10 may output the estimation result to the user by turning on a light emitter (illustration omitted). Alternatively, the estimation result may be output to the user by vibrations or may be output not only to the user but also to persons around the user (for example, the family of the user), or the like. As described above, in the present embodiment, by making such an output as described above enables the user to easily recognize that the physiological condition of the user is dehydration or anemia.
As described above, in the present embodiment, because a motion of the user that may lead to the blood flow amount decreased state in daily life is detected, it is possible to easily estimate a physiological condition of the user without requiring the user to execute a given motion. Accordingly, in the present embodiment, because the user is not required to execute a give motion, it is possible to avoid putting much strain on the user. Furthermore, according to the present embodiment, not only because putting much strain on the user is avoided but also because estimation is performed in daily life, it is also possible to easily collect data on the user regularly.

<2.7 Modification 1>

In the present embodiment described above, at step S103, a physical state of the user is detected based on the sensing data that is acquired from the motion recognition sensor 400 and, at step S105, whether the user corresponds to the blood flow amount decreased state is determined based on the detected physical state of the user. The present embodiment, however, is not limited to such a method and whether the user corresponds to the blood flow amount decreased state may be determined also with reference to the sensing data that is acquired from the blood flow sensor 300. This makes it possible to increase accuracy in determining whether the user corresponds to the blood flow amount decreased state. Such a modification will be described as Modification 1 of the first embodiment below.

Specifically, in the present modification, whether the user corresponds to the blood flow amount decreased state may be determined with reference to the shape of a pattern of change in the amount of blood flow over time based on the sensing data of the blood flow sensor 300. More specifically, the controller 100 may analyze the sensing data from the blood flow sensor 300 and determine whether the user corresponds to the blood flow amount decreased state based on whether such a pattern of change over time with a re-increase after a temporal drop like that illustrated n FIG. 6 can be detected. Furthermore, in Modification 1, whether the user corresponds to the blood flow amount decreased state may be determined also with reference to the sensing data of other various sensors that are arranged in the information processing device 10.

<2.8 Modification 2>

In the above-described embodiment, a physiological condition of the user is estimated based on a blood flow amount recovery slope at step S113. The present embodiment, however, is not limited to such a method, and a physiological condition of the user may be estimated also with reference to the context information described above. This makes it possible not only to increase accuracy in estimating a physiological condition of the user but also to detect a sign of change in the physiological condition (state in which change is highly likely to occur). Such a modification will be described below as Modification 2 of the first embodiment.

In Modification 2, a physiological condition of the user is estimated in consideration of not only the blood flow amount recovery slope but also, as context information, information, such as the weather (temperature, humidity, weather forecast), positional information (outdoor or indoor), and information, such as a schedule (exercise). For example, when it is known from the context information that the user is outdoor in high temperature, because the possibility of getting dehydrated is increasing, the lower limit of the given value range to be compared to the blood flow amount recovery slope that is calculated at step S109 is increased. This makes it possible not only increase accuracy in estimating dehydration of the user but also detect a sign leading to dehydration. In other words, according to Modification 2, it is possible to estimate a future physiological condition of the user that would lead to dehydration as early as possible. In Modification 2, a future physiological condition of the user may be estimated using machine learning to be described below.

<<3. Second Embodiment>>

In the first embodiment of the disclosure described above, a physiological condition of the user is estimated using the fact that the user enters the blood flow amount decreased state as a trigger and, in the second embodiment to be described below, an active stimulus is applied to a user and thus blood vessels are compressed to cause the physical state of the user to correspond to the blood flow amount decreased state. In the present embodiment, because this makes it possible to turn the physical state of the user into the blood flow amount decreased state regardless of motions of the user in daily life, it is possible to automatically estimate a physiological condition regularly without applying any strain on the user. Details of the present embodiment will be described in detail below sequentially.

<3.1 Functional Configuration of Information Processing Device 10>

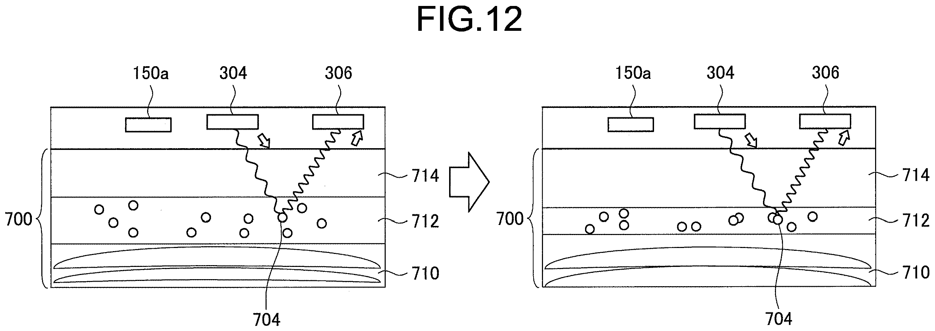

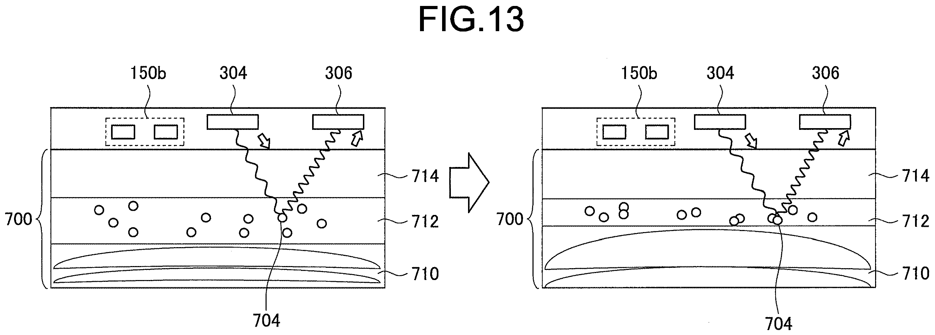

First of all, with reference to FIGS. 11 to 13, an example of a functional configuration of an information processing device 10a according to the present embodiment will be described. FIG. 11 is a block diagram illustrating the functional configuration of the information processing device 10a according to the present embodiment. FIGS. 12 and 13 are explanatory views for explaining a blood flow measurement method that is applied to the present embodiment.

As illustrated in FIG. 11, the information processing device 10a according to the embodiment includes, as in the first embodiment described above, for example, a controller 100a, the display unit 202, the blood flow sensor 300, and the motion recognition sensor 400. As illustrated in FIG. 11, the information processing device 10a according to the present embodiment further includes a stimulus generator (stimulator) 150. Thus, explanation of the functional units that are in common with the first embodiment will be omitted herein, only the stimulus generator 150 will be described, and details of the controller 100a will be described below.

(Stimulus generator 150)

The stimulus generator 150 is arranged on the inner side of the band unit 200 of the information processing device 10 and is able to apply a stimulus that pressurize blood vessels of the user (here, including a user wearing the information processing device 10 and another user wearing the information processing device 10), that is, a stimulus that changes the state of the blood vessels of the user to part of the body of the user. For example, the stimulus generator 150 consists of a cooling device 150a or an electrode 150b.

For example, when the stimulus generator 150 is the cooling device 150a like that illustrated in FIG. 12, it is possible to cause a blood vessel 712 to be contracted by cooling a surface of the skin 714 to make a blood vessel motion stimulus with the cooling device 150a and, as a result, it is possible to turn the physical state of the user into the blood flow amount decreased state.

For example, when the stimulus generator 150 includes electrodes 150b like those illustrated in FIG. 13, the blood vessel 712 is pressurized by applying a given voltage difference (electric stimulus) between the two electrodes 150b that are worn on the skin 714 to cause a muscle 710 to be contracted. As a result, it is possible to turn the physical sate of the user into the blood flow amount decreased state.

In the present embodiment, the stimulus generator 150 may be a pressure application device that causes the blood vessel 712 to be contracted by applying a pressure directly to part of the body of the user. In the present embodiment, the stimulus generator 150 may be a vibration device that applies vibrations to part of the body of the user. In this case, by performing blood flow measurement while applying vibrations, it is also possible to measure blood viscosity directly.

<3.2 Functional Configuration of Controller 100a>

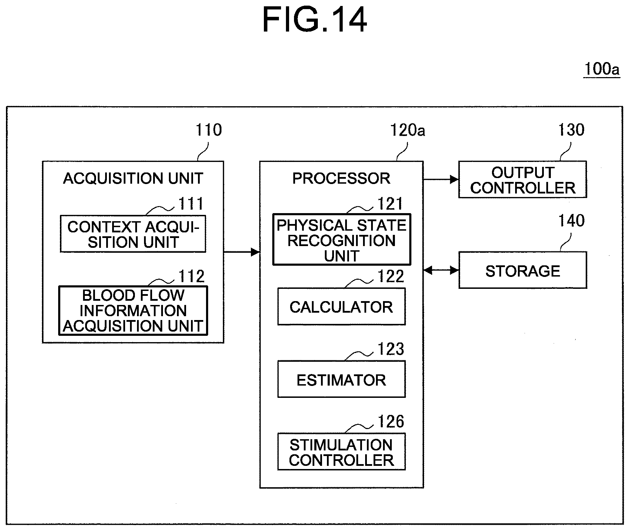

The example of the functional configuration of the information processing device 10a according to the present embodiment has been described. Subsequently, with reference to FIG. 14, an example of a functional configuration of the controller 100a of the information processing device 10 according to the present embodiment will be described. FIG. 14 is a block diagram illustrating the functional configuration of the controller 100 of the information processing device 10 according to the present embodiment. As illustrated in FIG. 14, the controller 100a of the information processing device 10a according to the present embodiment mainly includes, for example, as in the first embodiment, the acquisition unit 110, a processor 120a, the output controller 130, and the storage 140. Thus, explanation of the functions unit that are in common with the first embodiment will be omitted herein and only the processor 120a will be described.

(Processor 120a)

As in the processor 120 of the first embodiment, the processor 120a has a function of processing various types of information that are acquired by the acquisition unit 110 and outputting the processed information to the output controller 130 to be described below. Specifically, as illustrated in FIG. 14, the processor 120a includes the physical state recognition unit 121, the calculator 122, the estimator 123, and a stimulation controller 126. Explanation of the functional units that are in common with the first embodiment will be omitted herein and only the stimulation controller 126 will be described.

~Stimulation Controller 126~

The stimulation controller 126 is able to apply a given stimulus to part of the body of the user (including the user wearing the information processing device 10 and another user wearing the information processing device 10 herein) by controlling the stimulus generator 150. Note that change in the amount of blood flow over time that is obtained by applying a given stimulus to another user is stored as information that is used to estimate a physiological condition of the user.

<3.3 Information Processing Method>

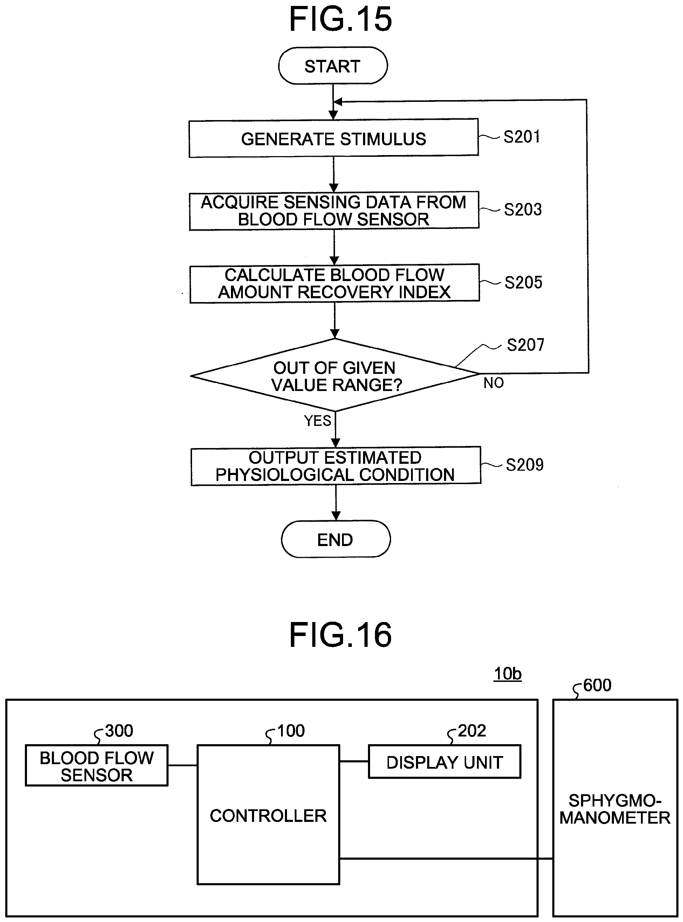

Next, with reference to FIG. 15, an information processing method according to the present embodiment will be described. FIG. 15 is a diagram illustrating a flowchart of the information processing method according to the present embodiment. As illustrated in FIG. 15, the information processing method according to the present embodiment includes multiple steps from step S201 to step S209. Each of the steps of the information processing method according to the present embodiment will be described below.

(Step S201)

First of all, by controlling the stimulus generator 150, the controller 100a applies a generated given stimulus to the body of the user. In the present embodiment, application of such a stimulus as described above turns the body of the user into the blood flow amount decreased state and an amount of blood flow is measured with the blood flow sensor 300.

(Steps S203 to S209)

Steps S203 to S209 illustrated in FIG. 15 are in common with steps S107 to S113 of the above-described first embodiment that are illustrated in FIG. 7 and thus description thereof will be omitted.

As described above, in the present embodiment, because applying the given stimulus makes it possible to turn the physical state of the user into the blood flow amount decreased state regardless of motions of the user in daily life, it is possible to automatically estimate a physiological condition regularly without applying any strain on the user.

<3.4 Modification>

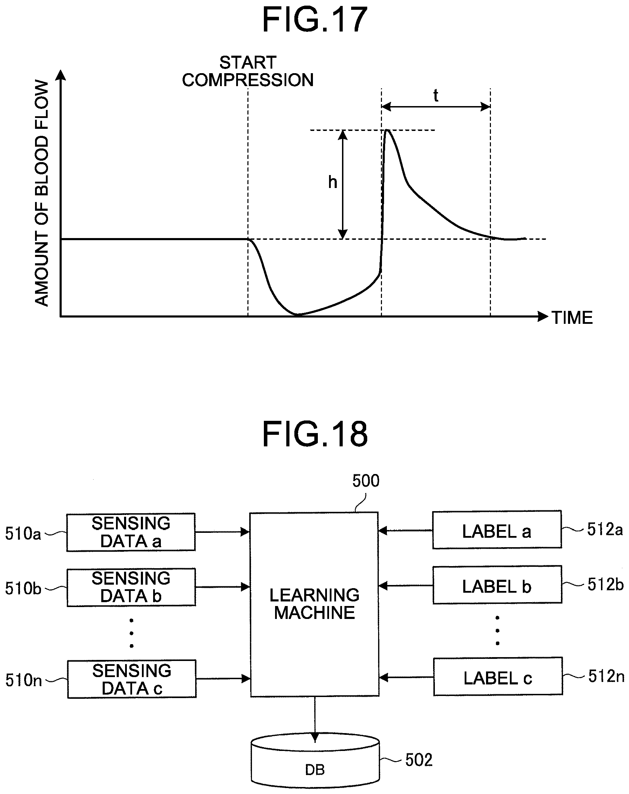

In the present embodiment, a sphygmomanometer capable of blocking a blood flow by applying a pressure to part of the body (specifically, an arm, or the like) of the user (here, including another user) is usable as the stimulus generator 150. The sphygmomanometer is capable of applying a pressure to part of the body of the user in every measurement under approximately the same condition, which makes it possible to obtain various types of information from change in the amount of blood flow over time that is obtained in such an optimum pressurized state. Details of such a modification of the present embodiment will be described below with reference to FIGS. 16 and 17. FIG. 16 is a block diagram illustrating a functional configuration of the information processing device 10a according to the modification of the present embodiment. FIG. 17 is an explanatory view for explaining the modification of the present embodiment and, specifically, illustrates change in the amount of blood flow over time.

As illustrated in FIG. 16, an information processing device 10b according to the present modification includes, as in the first embodiment described above, for example, the controller 100, the display unit 202, and the blood flow sensor 300. As illustrated in FIG. 16, the information processing device 10b according to the present embodiment is connected to a hemodromometer 600 such that they are able to communicate with each other and, for example, the information processing device 10b controls the hemodromometer 600 and thus is able to operate in association with the hemodromometer 600. The sphygmomanometer 600 is a device that calculates a systolic blood pressure and a diastolic blood pressure in a way that a pressurizing device referred to as a cuff is wound around part of the body of the user (for example, an upper arm or a wrist) and the cuff pressurizes part of the body to block the blood flow and then decompresses the part of the body gradually and change in the mode of blood flow in the artery corresponding to the relationship in magnitude between the value of pressurization and the minimum blood pressure is detected. The blood pressure herein refers to a pressure by which the flow of blood pumped from the heart pushes the blood vessel from the inside and is expressed by two numeric values of a blood pressure at contraction of the heart (maximum pressure) and a blood pressure at relaxation of the heart (minimum pressure). Note that, in the present modification, the hemodromometer 600 is not used to measure a blood pressure but is usable to block the blood flow of the user, that is, create the blood flow amount decreased state and attach a label (event detection) indicating that blood flow information that is acquired by the blood flow sensor 300 is data in the blood flow amount decreased state to the blood flow information. It is described above that the blood flow is blocked with the cuff but the present modification is not limited to such a cuff and another pressurizing device other than the cuff may be used as long as the pressurizing device is capable of blocking the blood flow.

In the present modification, each functional block (the controller 100, the display unit 202, and the blood flow sensor 300) of the information processing device 10b is the same as that of the first embodiment and thus description thereof will be omitted herein.

Details of the present modification will be described below with reference to FIG. 17 and, according to the following description, the hemodromometer 600 is worn on an upper arm of the user and the information processing device 10b (specifically, the blood flow sensor 300) is worn around a wrist of the user.

First of all, the sphygmomanometer 600 pressurizes the upper arm by a pressure equal to or higher than the maximum pressure, so that, as illustrated in FIG. 17, the amount of skin blood flow in the wrist drops to a value close to 0, that is, the arterial blood flow is blocked. Thereafter, the sphygmomanometer 600 performs decompression after keeping pressurization for a given time (for example, few tens of seconds) and the primary loss of the arterial blood flow by compression causes the physiological system of the body of the user to expand the blood vessel in order to recover the blood flow state and increases the amount of blood flow as illustrated in FIG. 17.

Such a behavior of increase in the amount of blood flow is, for example, representable by an index, such as an amount (h in FIG. 17) of an overshoot of the amount of blood flow with respect to a steady amount of blood flow (before the measurement) or a length of elapse of time until recovery to the steady amount of the blood flow after the overshoot. In the field of medicine, such an index of increase in the amount of blood flow after compression is referred to as a Post-Occlusive forearm skin Reactive Hyperaemia (PORH) index. It is considered that a PORH index reflects the condition of a vascular function and a cardiac function of the user and, for example, it is known that the rate of increase in the amount of blood flow (for example, h in FIG. 17) is small when vascular dysfunction occurs locally due to diabetes.

The present modification is not limited to such estimation of dehydration and anemia as that described above and it is also possible to, using a PORH index, etc., estimate the condition of the vascular function, the cardiac function and the autonomic nervous system. More specifically, in the present modification, the sphygmomanometer 600 compresses the upper arm by a pressure equal to or higher than the maximum blood pressure and thereafter decompresses the upper arm and the blood flow sensor 300 detects change in the amount of blood flow of the wrist of the user during that time. The information processing device 10b calculates an overshoot amount (h), a length of elapse of time (t), or the like, which is described above, from the change in the amount of blood flow and, based on the calculated index, estimates a physiological condition (health condition) of the user in the vascular function, the cardia function, the autonomic nervous system, and a disease (such as diabetes).

In the present modification, blocking the blood flow using the sphygmomanometer 600 makes it possible to create the given blood flow amount decreased state every time and thus it is possible to compare sets of blood flow information in approximately the same states, which enables an increase in accuracy in estimating a physiological condition of the user. Furthermore, in the present modification, because it is possible to perform an estimation using an index with a lot of medical findings, such as a PORH index, it is possible to perform not only estimation of dehydration and anemia but also various estimations of the vascular function, the cardiac function, and the autonomic nervous system of the user.

The present modification is not limited to using a PORH index, and a physiological condition of the user may be estimated using another index. For example, a physiological condition of a user may be estimated using, as an index, a shape of a peak appearing in change in the amount of blood flow of the user over time (waveform), an integral of amounts of blood flow in a given time, or the like. Furthermore, in the present embodiment, for example, a database obtained by performing machine learning on multiple changes in the amount of blood flow of the user or another user that were acquired in the past and that are labelled (labelled with profile information, physiological conditions, or the like) may be referred to.

Note that, according to the description above, the information processing device 10b and the hemodromometer 600 are connected such that they are able to communicate with each other but the present modification is not limited to connecting the information processing device 10b and the hemodromometer 600 such that they are able to communicate with each other and they need not be connected with each other. In this case, the information processing device 10b is able to previously perform machine learning on change in the amount of blood flow over time (waveform) caused by pressurization with the hemodromometer 600 and automatically detect the blood flow amount decreased state with reference to a feature value obtained by machine learning and based on newly acquired change in the amount of blood flow over time.

The present modification is not limited to the hemodromometer 600 as long as the blood flow of the user can be blocked and, for example, an occlusion tool that is used in occlusion training may be used. Occlusion training is a training method in which an arm or the groin is preferably pressurized with a belt-like dedicated occlusion tool and training is performed in a state where the blood flow is preferably restricted.

<<4. Third Embodiment>>

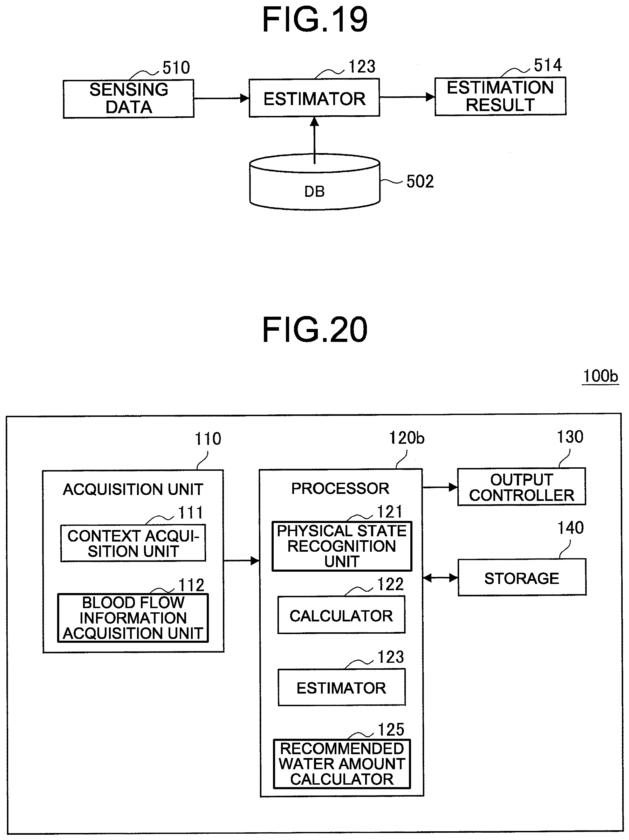

Note that the tendency of recovery of the amount of blood flow differs depending on the individual. Thus, in a third embodiment of the disclosure, different from each of the embodiments described above, a database (DB) of each user is created by machine learning and a physiological condition is estimated based on the DB. According to the present embodiment, this enables estimation dedicated to each individual and thus enables more accurate estimation of a physiological condition. With reference to FIG. 18 and FIG. 19, the present embodiment using machine learning will be described below sequentially as the third embodiment of the disclosure.

In the present embodiment, for example, a learning machine is arranged in the information processing device 10 in order to perform machine learning. Specifically, a supervised learning machine 500 of support vector regression, deep neural network, or the like, is arranged in the information processing device 10. As illustrated in FIG. 18, multiple sets of sensing data 510 of a specific user that are acquired from the blood flow sensor 300 and results (labels) 512 of estimating a physiological condition of the user corresponding to the sets of sensing data 510 are input as input signals and training signals, respectively, to the learning machine 500 and the learning machine 500 performs machine learning on the relation among these sets of information according to given rules. Pairs of the aforementioned input signals and the training signals are input to the learning machine 500 and the learning machine 500 performs machine learning on these inputs, thereby constructing a database (DB) 502 that stores relationship information indicating the relationship between the sensing data 510 and the estimation result (label) 512 is constructed.

More specifically, in the present embodiment, for example, it is estimated that the user is after a meal from a motion of the user (such as a motion of drinking) that is estimated based on sensing data obtained by an acceleration sensor (illustration omitted) that is worn around an arm, a position (cafeteria) that is estimated based on sensing data obtained by a positioning sensor (illustration omitted), and a schedule (breakfast time) that is estimated based on time. In the present embodiment, when such an estimation is made, it is supposed that the user is in a state where water is taken sufficiently and the blood viscosity is in a steady state (normal state). Furthermore, in the present embodiment, the sensing data 510 from the blood flow sensor 300 that is acquired at that time is input in association with the label 512 of the steady state to the learning machine 500.

In the present embodiment, for example, when it is estimated that the user is doing a hard exercise based on the sensing data obtained by the acceleration sensor, the sensing data 510 from the blood flow sensor 300 that is acquired at that time is input in association with the label 512 of dehydration to the learning machine 500. Furthermore, in the present embodiment, for example, when it is estimated that the user has been in an upright state for a long time based on the sensing data obtained by, for example, the acceleration sensor and a barometric pressure sensor, the sensing data 510 from the blood flow sensor 300 that is acquired at that time is input in association with the label 512 of anemia to the learning machine 500.

In the present embodiment, it is preferable to label the sensing data 510, keeping in mind the following aspects. Generally, anemia tends to be noticed because of occurrence of dizziness and symptoms of palpitations, shortness of breath, being easily fatigued, fatigue, etc., also tend to appear. In the present embodiment, when the fact that move of the user is slow and the time in which the user lies gets longer is captured with the acceleration sensor (illustration omitted), the label of anemia may be attached. In the present embodiment, the blood flow sensor 300 makes it possible to capture palpitations from an increase in the pulse rate (a heart rate is calculated from the blood flow pulse wave) and capture breathlessness from an increase in the respiration rate (a respiration rate is estimated from fluctuations in the amplitude of the blood flow pulse wave).

In the present embodiment, a semi-trained learning machine or a weakly supervised learning machine may be used for the learning machine 500.

Furthermore, as illustrated in FIG. 19, based on the DB 502 that is obtained by machine learning by the learning machine 500, it is possible to newly estimate a physiological condition of the user (estimation result) 514 from the sensing data 510 that is newly acquired from the blood flow sensor 300.

As described above, in the present embodiment, because a database (DB) of each user is created using machine learning and a physiological condition is estimated based on the DB, it is possible to make an estimation dedicated to each individual and estimate a physiological condition more accurately.

Machine learning like that described above is applicable not only to estimation of a physiological condition but also to detection of a physical state of the user based on sensing data from the motion recognition sensor 400 in the first embodiment.

In the present embodiment, the above-described DB may be created by also performing machine learning on multiple changes in the amount of blood flow of another user that were acquired in the past and that are labelled (labelled with profile information, physiological conditions, or the like).

<<5. Fourth Embodiment>>

In a fourth embodiment of the disclosure to be described below, an amount of water taken by the user is estimated from a blood flow amount recovery index (blood flow amount recovery slope). The amount of fluid in blood increase as the user takes water and thus blood viscosity decreases and therefore the amount of blood flow recovers fast. Accordingly, in the present embodiment, utilizing such a mechanism, it is possible to estimate an amount of water that is taken by a user from a tendency of recovery of the amount of blood flow. Furthermore, in the present embodiment, an amount of water that is recommended to the user (recommended water intake amount) is calculated based on the estimated amount of water and the recommended water intake amount is output to the user. According to such an embodiment, because it is possible to recommend a preferable amount of water to the user, it is possible to support maintaining the physiological condition of the user preferably. Details of the present embodiment will be described below sequentially.

<5.1 Functional Configuration of Controller 100a>

First of all, with reference to FIG. 20, an example of a functional configuration of a controller 100b of the information processing device 10 according to the present embodiment will be described. FIG. 20 is a block diagram illustrating the functional configuration of the controller 100b of the information processing device 10 according to the present embodiment. As illustrated in FIG. 20, the controller 100b of the information processing device 10 according to the present embodiment mainly includes, for example, as in the first embodiment, the acquisition unit 110, a processor 120b, the output controller 130, and the storage 140. Thus, explanation of the function units that are in common with the first embodiment will be omitted herein and only the processor 120b will be described.

(Processor 120b)

As in the processor 120 of the first embodiment, the processor 120b has a function of processing various types of information that are acquired by the acquisition unit 110 and outputting the processed information to the output controller 130 to be described below. Specifically, as illustrated in FIG. 20, the processor 120b includes the physical state recognition unit 121, the calculator 122, the estimator 123, and a recommended water amount calculator 125. Explanation of the functional units that are in common with the first embodiment will be omitted herein and only the recommended water amount calculator 125 will be described.

~Recommended Water Amount Calculator 125~

The recommended water amount calculator 125 estimates an amount of water that is taken by the user from a blood flow amount recovery slope and, based on the estimated amount of water, calculates an amount of water that is recommended to the user. Specifically, the recommended water amount calculator 125 incorporates the learning machine 500 that creates the DB 502 indicating a relationship between the amount of water taken and the blood flow amount recovery slope, utilizing the above-described machine learning. Note that, in the present embodiment, the relationship between the amount of water taken and the blood flow amount recovery slope may be derived by performing not only machine learning but also statistic processing.

<5.2 Information Processing Method>

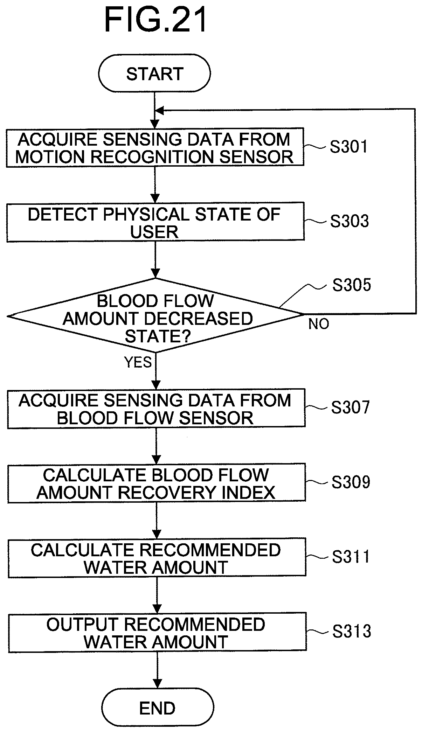

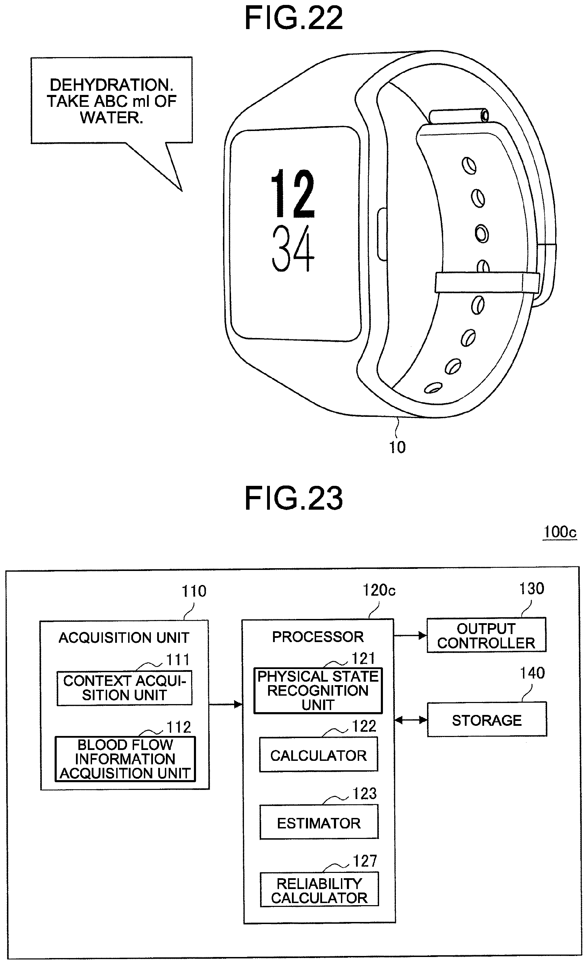

With reference to FIGS. 21 and 22, the information processing method according to the present embodiment will be described next. FIG. 21 is a flowchart illustrating an example of the information processing method according to the present embodiment and FIG. 22 is an explanatory view illustrating an example of output of an estimation result according to the present embodiment. As illustrated in FIG. 21, the information processing method according to the present embodiment includes multiple steps from step S301 to step S313. Each of the steps of the information processing method according to the present embodiment will be described below.

(Steps S301 to S309)

Steps S301 to step S309 illustrated in FIG. 21 are in common with steps S101 to S109 of the first embodiment described above that are illustrated in FIG. 7 and thus description thereof will be omitted.

(Step S311)

The controller 100b estimates an amount of water that is taken by the user based on a blood flow amount recovery index (blood flow amount recovery slope) that is calculated at step S309. The controller 100b further calculates a recommended water amount (estimation result) that is recommended to the user based on the estimated amount of water taken by the user.

(Step S313)

The controller 100b outputs the recommended water amount that is calculated at step S311 described above to the user. For example, as illustrated in FIG. 22, the information processing device 10 may output the estimation result to the user by outputting a voice "Dehydration. Take ABC ml of water." via a speaker (illustration omitted). The controller 100b then ends the process.

As described above, according to the present embodiment, because it is possible to recommend a preferable amount of water to the user, it is possible to assist in maintaining the physiological condition of the user preferably. Note that the present embodiment may be carried out in combination with the second embodiment in which an active stimulus is applied to the user.

In the present embodiment, to estimate an amount of water that is taken by the user, a motion of lifting a glass by the user on which estimation is performed may be detected based on the sensing data obtained by an acceleration sensor (illustration omitted) that is worn around an arm or a throat (around the neck) and move of the throat of the user may be detected. This makes it possible to increase accuracy in estimating an amount of water that is taken.

## <<6. Fifth Embodiment>>

In a fifth embodiment of the disclosure to be described below, a reliability of estimation of a physiological condition of the user, which is described above, is calculated and the reliability is output to the user. In the present embodiment, outputting the reliability of the result of estimating a physiological condition to the user enables the user to determine a behavior (taking water) that should be taken next based on the reliability of the estimated physiological condition. Details of such an embodiment will be described below sequentially.

### <6.1 Method of Calculating Reliability>

In the present embodiment, as a method of estimating a reliability, for example, a method using a degree of deviation from a steady (normal) blood flow amount recovery slope as reliability is taken.
More specifically, in dehydration, when the steady (normal) blood flow amount recovery slope is a, a blood flow amount recovery slope that is newly obtained is b (b<a), and a lower limit of a given value range is c, a reliability R is representable by Equation (4) below.

$$R = \frac{x - a}{c - a} \qquad (4)$$

where, x = b when b $\leq$ c, and x = c when b > c.
As can be seen from Equation (4), the closer to the lower limit c in the given value range the blood flow amount recovery slope b is, the closer to 1 the reliability R is, which means that the reliability R is high.

### <6.2 Functional Configuration of Controller 100c>

The example of the method of calculating a reliability R according to the present embodiment has been described above. Subsequently, with reference to FIG. 23, an example of a functional configuration of a controller 100c of the information processing device 10 according to the present embodiment will be described. FIG. 23 is a block diagram illustrating the functional configuration of the controller 100c of the information processing device 10 according to the present embodiment. As illustrated in FIG. 23, for example, as in the first embodiment, the controller 100c of the information processing device 10 according to the present embodiment mainly includes the acquisition unit 110, a processor 120c, the output controller 130 and the storage 140. Thus, description of the functional units that are in common with the first embodiment will be omitted herein and only the processor 120c will be described.

### (Processor 120c)

As in the processor 120 of the first embodiment, the processor 120c has a function of processing various types of information that are acquired by the acquisition unit 110 and outputting the processed information to the output controller 130 to be described below. Specifically, as illustrated in FIG. 23, the processor 120a includes the physical state recognition unit 121, the calculator 122, the estimator 123, and a reliability calculator 127. Explanation of the functional units that are in common with the first embodiment will be omitted herein and only the reliability calculator 127 will be described.

### ~Reliability Calculator 127~

The reliability calculator 127 calculates a reliability of a result of estimating a physiological condition of the user based on a deviation degree indicating a degree (amount) of deviation from the steady (normal) blood flow amount recovery slope.

### <6.3 Information Processing Method>

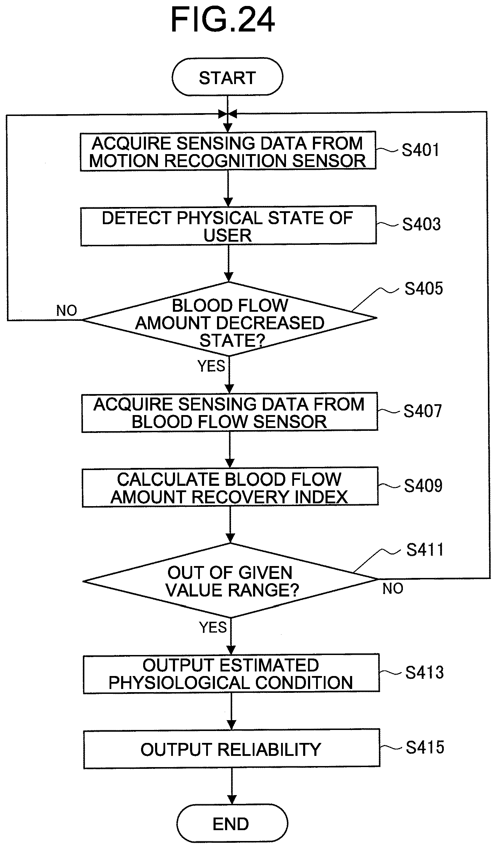

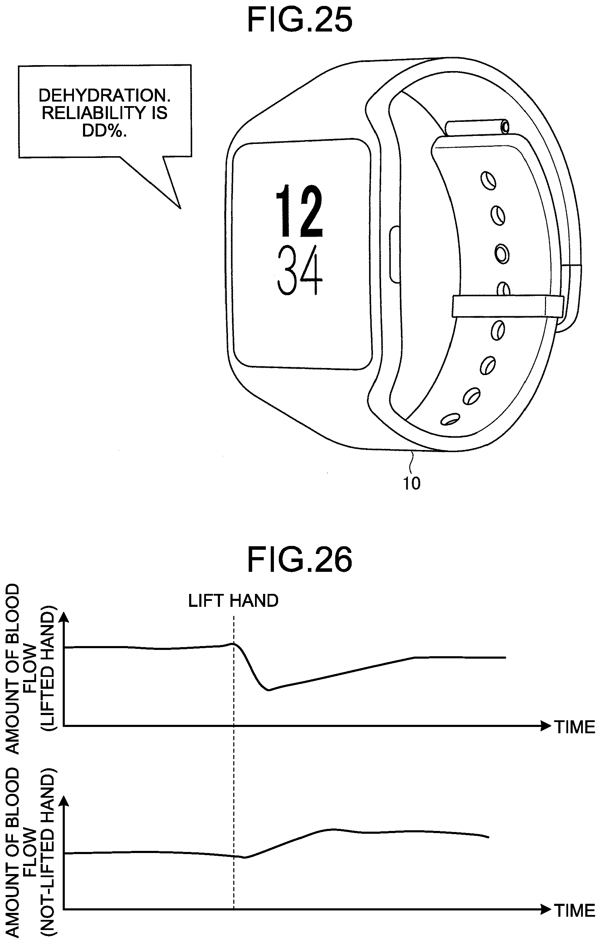

With reference to FIGS. 24 and 25, the information processing method according to the embodiment will be described. FIG. 24 is a diagram illustrating a flowchart of the information processing method according to the present embodiment and FIG. 25 is an explanatory view illustrating an example of output of an estimation result according to the present embodiment. As illustrated in FIG. 24, the information processing method according to the present embodiment includes multiple steps from step S401 to step S415. Each of the steps of the information processing method according to the present embodiment will be described below.

(Steps S401 to S413)

Steps S401 to step S413 illustrated in FIG. 24 are in common with steps S101 to S113 of the above-described first embodiment that are illustrated in FIG. 7 and thus description thereof will be omitted.

(Step S415)

The controller 100c calculates a reliability R of the physiological condition of the user that is estimated at step S413 and outputs the reliability R to the user. For example, as illustrated in FIG. 25, the information processing device 10 may output the estimation result to the user by outputting a voice "Dehydration. Reliability is DD%." via a speaker (illustration omitted). The controller 100c then ends the process.

As described below, in the present embodiment, a reliability R of estimation of a physiological condition of the user, which is described above, is calculated and the reliability R is output to the user and therefore the user is able to determine a behavior that should be taken next (taking water) based on the reliability R of the estimated physiological condition.

In the present embodiment, the time and frequency of sensing performed by the blood flow sensor 300 for the next time may be changed based on the calculated reliability R. Furthermore, the present embodiment may be carried out in combination with the second embodiment in which an active stimulus is applied to the user.

<<7. Sixth Embodiment>>

In the embodiments described above, the information processing device 10 (specifically, the blood flow sensor 300) has been described as one that is worn on one part of the body of the user; however, the embodiments of the disclosure are not limited thereto. In the embodiments of the disclosure, for example, the information processing devices 10 may be worn on multiple parts of the body of one user that are different from each other and acquire sets of blood flow information on the multiple parts (for example, first and second blood flow amount changes). With reference to FIG. 26, such an embodiment will be described as a sixth embodiment of the disclosure. FIG. 26 is an explanatory view for explaining the present embodiment and, specifically, the upper chart represents change in the amount of blood flow over time on the side of a hand that the user lifts and the lower chart represents change in the amount of blood flow over time on the side of the hand that the user does not lift.

In the following description, for example, the information processing device 10 according to the first embodiment described above is worn on two parts of the user that are the right wrist and the left wrist. In this case, when the user lifts one of the hands above the heart of the user, as illustrated in the upper chart in FIG. 26, the arm enters the blood flow amount decreased state and accordingly the amount of blood flow decreases temporarily (local response). When the user is healthy, a function of pumping blood flow by increasing the blood pressure works in order to take balance of blood flow over the body and the amount of blood flow recovers to the state before the lift of the hand (general response). At that time, because, as illustrated in the lower chart of FIG. 26, the arm of the other hand that the user does not lift above the heart is not in the blood flow amount decreased state, a behavior that the amount of blood flow decreases temporarily does not appear. In the arm, however, the blood pressure increases because the other hand is lifted above the heart and thus the amount of blood flow gets higher than that in the state before the lift of the arm (general response).

In the present embodiment, by detecting change in the amount of blood flow over time in such multiple parts, blood flow information regarding local responses of the multiple parts of the body of the user or a combination of sets of blood flow information regarding local responses and the general response are acquired. In the present embodiment, the information processing device 10 thus estimates a physiological condition of the user (for example, dehydration, anemia, the vascular function, the cardiac function, and the autonomic nervous system) by, for example, comparing the sets of blood flow information that are acquired as described above (for example, comparing the sets of blood flow information regarding the local responses and the blood flow information regarding the general response) and making an analysis on such a combination of sets of blood flow information resulting from the responses of the multiple parts (for example, calculating an index from the combination or comparing the combination with a combination of another user, etc.).

As described above, in the present embodiment, by detecting such change in amounts of blood flow over time in multiple parts, it is possible to acquire blood flow information regarding local responses of the parts of the body of the user or a combination of sets of blood flow information regarding local responses and a general response. According to the present embodiment, estimating a physiological condition of the user based on such a combination of sets of blood flow information resulting from responses of the multiple parts makes it possible to increase accuracy of estimation. Furthermore, according to the present embodiment, such a combination of sets of blood flow information resulting from responses of multiple parts does not put limitation to estimation of dehydration, anemia, etc., it is possible to make various estimations on the vascular function, cardiac function, the autonomic nervous system, etc.

According to the present embodiment, the number of parts of measurement are not limited to two and more than two parts may apply and the part is not limited to the wrist. In the present embodiment, the information processing devices 10 are not limited to wristwatch-type wearable devices and, for example, a wearable device and a device in a mode of an imaging device capable of acquiring the above-described speckle pattern may be used.

<<8. Summary>>

As described, according to each of the embodiments of the disclosure, because such a motion of a user as one leading to the blood flow amount decreased state is detected in daily life and an active stimulation is applied to the user to cause the user to enter that state, it is possible to easily estimate a physiological condition of the user without requiring the user to execute a given motion. Accordingly, in the present embodiment, because the user is not required to execute a given motion, it is possible to avoid putting much strain on the user. In other words, according to each of the embodiments of the disclosure, it is possible to easily estimate a physiological condition of the user in motions in daily life. Furthermore, according to the present embodiment, because the information processing device makes an estimation in daily life in addition to avoiding putting much strain on the user, it is possible to easily collect data on the user regularly.

The information processing device 10 according to the present embodiment may be applied to a system consisting of multiple devices based on connection to a network (or communication between devices) as in, for example, cloud computing. In other words, it is possible to realize the information processing device 10 according to the above-described embodiment, for example, as an information processing system that performs a process according to the information processing method according to the present embodiment using multiple devices.

For example, the controller 100 of the information processing device 10 according to the embodiment may be used in an information processing system in which the acquisition unit 110 is installed in a wearable device and the processor 120, etc., are installed in a cloud server. In general, because the rate at which a cloud server processes data is higher than the rate at which a wearable device processes data, such an information processing system as that described above, for example, makes it possible to increase the rate at which a physiological condition of the user is estimated. Furthermore, this makes it possible to inhibit the power consumption of the wearable device from increasing.

<<9. Hardware Configuration>>

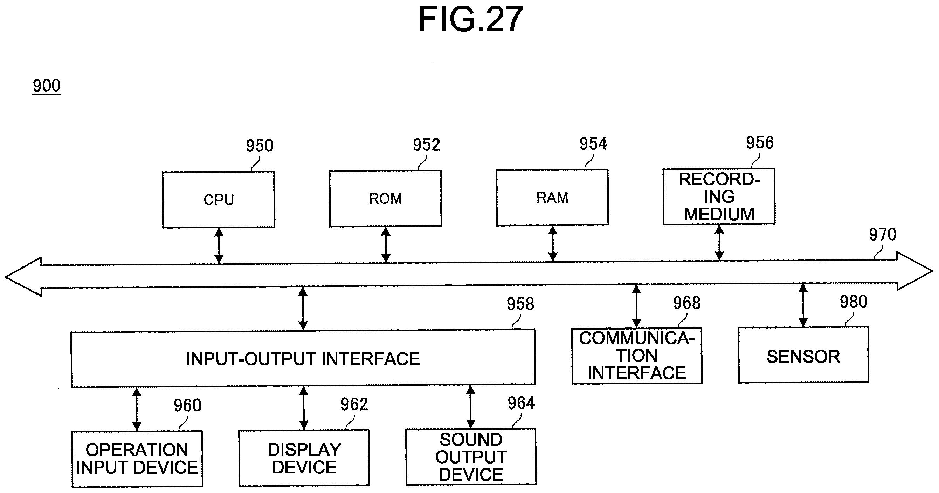

FIG. 27 is an explanatory view illustrating an example of a hardware configuration of an information processing device 900 according to the present embodiment. In FIG. 27, the information processing device 900 represents an example of a hardware configuration of the information processing device 10 described above.

The information processing device 900 includes, for example, a CPU 950, a ROM 952, a RAM 954, a recording medium 956, an input-output interface 958, and an operation input device 960. The information processing device 900 further includes a display device 962, a sound output device 964, a communication interface 968, and a sensor 980. In the information processing device 900, for example, components are connected via a bus 970 serving as a data transmission path.

(CPU 950)

The CPU 950 consists of at least one processor consisting of an operation circuit, such as a CPU, and various processing circuits and is capable of functioning as the controller 100 that generally controls the information processing device 900.

(ROM 952 and RAM 954)

The ROM 952 stores control data, such as programs and operation parameters that are used by the CPU 950. The RAM 954 temporarily stores, for example, programs that are executed by the CPU 950, etc. The ROM 952 and the RAM 954, for example, function as the storage 140 described above in the information processing device 900.

(Recording Medium 956)

The recording medium 956 functions as the storage 140 described above and stores, for example, various types of data, such as data regarding the information processing method according to the embodiment and various types of data, such as various applications.

As the recording medium 956, for example, a magnetic recording medium, such as a hard disk, or a non-volatile memory, such as a flash memory, is taken. The recording medium 956 may be detachable from the information processing device 900.

(Input-output Interface 958, Operation Input Device 960 and Display Device 962)

The input-output interface 958, for example, connects the operation input device 960, the display device 962, etc. As the input-output interface 958, for example, a USB (Universal Serial Bus) terminal, a DVI (Digital Visual Interface) terminal, a HDMI (High-Definition Multimedia Interface) (trademark) terminal, or various processing circuits are taken.

The operation input device 960 functions as an operation unit (illustration omitted) and, for example, is included by the information processing device 900 and is connected to the input-output interface 958 in the information processing device 900. As the operation input device 960, for example, a keyboard, a button, an arrow key, a rotary selector, such as a jog dial, a touch panel or a combination thereof is taken.

The display device 962 functions as an information presentation device consisting of the display unit 202 described above, for example, is on the information processing device 900, and is connected to the input-output interface 958 in the information processing device 900. As the display device 962, for example, a liquid crystal display or an organic EL (electro-luminescence) display is taken.

Needless to say, the input-output interface 958 is connectable to external devices, such as an operation input device (for example, a keyboard or a mouse) or an external display device outside the information processing device 900.

(Communication Interface 968)

The communication interface 968 is a communication unit that the information processing device 900 includes and functions as a communication unit (illustration omitted) for communicating with an external device, such as a server, in a wired or wireless manner via a network (or directly). As the communication interface 968, for example, a communication antenna and an RF (Radio Frequency) circuit (wireless communication), an IEEE802.15.1 port and a transmitter-receiver circuit (wireless communication), an IEEE802.11 port and a transmitter-receiver circuit (wireless communication), or a LAN (Local Area Network) terminal and a transmitter-receiver circuit (wired communication) is taken.

(Sensor 980)

The sensor 980 functions as the blood flow sensor 300 and the motion recognition sensor 400 and, for example, is a sensor with any system enabling detection of blood flow information of the user, etc. The sensor 980 may include, as the above-described motion recognition sensor 400, at least one sensor, such as an acceleration sensor or a gyro sensor. In other words, the sensor included by the sensor 980 is not limited to the above-described example.

The hardware configuration of the information processing device 900 is not limited to the configuration illustrated in FIG. 27. For example, the information processing device 900 need not include the communication interface 968 when communicating with an external device, or the like, via an external communication device to which the information processing device 900 is connected or when being configured to perform stand-alone processing. The communication interface 968 may have a configuration enabling communication with at least one external device using multiple communication systems. The information processing device 900, for example, may be configured without the recording medium 956, the operation input device 960, the display device 962, etc.

The information processing device 900 has been taken and described as the present embodiment but the present embodiment is not limited to the mode. The present embodiment is, for example, applicable to various devices capable of performing processing according to the information processing method according to the present embodiment, such as a communication device like a mobile phone.

The information processing device 900 according to the present embodiment, for example, may be applied to a system consisting of multiple devices based on connection to a network (or communication between devices) as in cloud computing, or the like. In other words, for example, it is possible to realize the information processing device 900 according to the embodiment described above as an information processing system that performs a process according to the information processing method according to the present embodiment using multiple devices.

The example of the hardware configuration of the information processing device 900 has been presented above. Each of the above-described components may be configured using general-purpose members or may be configured of hardware dedicated to the function of each component. The configuration is changeable appropriately according to the technical level of each implementation.

<<10. Supplementary>>

The embodiments of the disclosure described above can include a program for causing a computer to function as the information processing device according to the present embodiment and a non-temporary and tangible medium in which the program is recorded. The program may be distributed via a communication line (including wireless communication).

Each step of the process of each of the embodiments described above need not necessarily be processed along the described order. For example, each step may be processed in an order that is changed appropriately. Each step may be processed partly in parallel or individually instead of being processed chronologically. Furthermore, as for the processing method of each step, processing need not necessarily be performed along the described method and, for example, processing may be performed by another method by another functional unit.

The preferable embodiments of the disclosure have been described in detail with reference to the accompanying drawings; however, the technical scope of the disclosure is not limited to the examples. It is obvious that those with general knowledge in the technical field of the disclosure can reach various modifications or corrections within the scope of the technical idea described in the claims and it is understood that they naturally belong to the technical scope of the disclosure.

The effects described herein are explanatory or exemplary only and thus are not definitive. In other words, the technique according to the disclosure can achieve, together with the above-described effects or instead of the above-described effects, other effects obvious to those skilled in the art from the description herein.

The following configuration also belongs to the technical scope of the disclosure.

(1) An information processing device comprising a physiological condition estimator configured to estimate a physiological condition of a user who is in a given physical state based on a first blood flow amount change of the user and a second blood flow amount change of the user or another user in the given physical state, the first blood flow amount change and the second blood flow amount change being acquired by a blood flow information detector.

(2) The information processing device according to (1), wherein the second blood flow amount change is acquired as a reference value previously.

(3) The information processing device according to (2), further comprising a state detector configured to detect that the user or the another user is in the given physical state by detecting a given motion of the user or the another user in daily motions.

(4) The information processing device according to (3), wherein the state detector is configured to detect that the user or the another user is in the given physical state based on sensing data that is acquired by the blood flow information detector.

(5) The information processing device according to (2), further comprising a stimulation controller configured to control a stimulator configured to apply a stimulus that changes a state of a blood vessel of the user or the another user to a body of the user or the another user,
wherein, when the stimulus applied by the stimulator leads to the given physical state, the first blood flow amount change and the second blood flow amount change are acquired by the blood flow information detector.

(6) The information processing device according to (5), wherein the stimulator is configured to apply the stimulus that cools a skin surface of the user or the another user to the user or the another user.

(7) The information processing device according to (5), wherein the stimulator is configured to apply the stimulus that squeezes a muscle of the user or the another user to the user or the another user.

(8) The information processing device according to (5), wherein the stimulator is configured to apply a pressure that blocks a blood flow of the user or the another user.

(9) The information processing device according to (8), wherein the stimulator is a sphygmomanometer.

(10) The information processing device according to any one of (1) to (7), wherein the given physical state is a state in which a blood vessel of the user or the another user is pressurized or a state in which the blood vessel of the user or the another user is contracted.

(11) The information processing device according to any one of to (1) to (10), wherein the physiological condition estimator is configured to estimate a future physiological condition of the user based on the first blood flow amount change and the second blood flow amount change.

(12) The information processing device according to (3), further comprising a context acquisition unit configured to acquire context information on the user or the another user,
wherein the state detector is configured to detect that the user or the another user is in a given physical state based on the context information.

(13) The information processing device according to any one of (1) to (12), further comprising an output unit configured to output an estimation result of the estimated physiological condition of the user.

(14) The information processing device according to any one of (1) to (13), wherein a blood flow amount recovery slope is used as the first and second blood flow amount changes, the blood flow amount recovery slope being a slant of an amount of blood flow of the user or the another user with respect to time and being obtained after the amount of blood flow turns to an increase after temporarily decreasing because the user or the another user enters the given physical state.

(15) The information processing device according to (14), wherein the blood flow recovery slope is a slant that is obtained by dividing a sum of amounts of blood flow until elapse of a given time after the increase of the amount of blood flow by the given time.

(16) The information processing device according to (14) or (15), wherein the physiological condition estimator is configured to estimate that the user has dehydration when a first blood flow amount recovery slope that is the first blood flow amount change is smaller than a reference vale that is obtained from a second blood flow amount recovery slope that is the second blood flow amount change.

(17) The information processing device according to (16), further comprising a recommended water amount calculator configured to,

when it is estimated that the user has dehydration, estimate an amount of water that is taken by the user based on the first blood flow amount recovery slope and
estimate a recommended water intake amount that is an amount of water that is recommended to take to the user based on the estimated amount of water that is taken.

(18) The information processing device according to (14) or (15), wherein the physiological condition estimator is configured to estimate that the user has anemia when a first blood flow amount recovery slope that is the first blood flow amount change is larger than a reference vale that is obtained from a second blood flow amount recovery slope that is the second blood flow amount change.

(19) The information processing device according to (16), further comprising reliability calculator configured to calculate a reliability of a result of estimating the physiological state of the user based on a degree of deviation indicating a degree at which the first blood flow amount recovery slope deviates from the second blood flow amount recovery slope.

(20) The information processing device according to (1), wherein the first blood flow amount change and the second blood flow amount change are acquired by the blood flow information detector that is worn on parts of a body of the user, the parts being different from each other.

(21) An Information processing method comprising estimating a physiological condition of a user who is in a given physical state based on a first blood flow amount change of the user and a second blood flow amount change of the user or another user in the given physical state, the first blood flow amount change and the second blood flow amount change being acquired by a blood flow information detector.

(22) An program for causing a computer to implement a function of estimating a physiological condition of a user who is in a given physical state based on a first blood flow amount change of the user and a second blood flow amount change of the user or another user in the given physical state, the first blood flow amount change and the second blood flow amount change being acquired by a blood flow information detector.

Reference Signs List

| | |
|---|---|
| 10, 10a, 10b, 900 | INFORMATION PROCESSING DEVICE |
| 100, 100a, 100b, 302 | CONTROLLER |
| 110 | ACQUISITION UNIT |
| 111 | CONTEXT ACQUISITION UNIT |
| 112 | BLOOD FLOW INFORMATION ACQUISITION UNIT |
| 120, 120a, 120b | PROCESSOR |
| 121 | PHYSICAL STATE RECOGNITION UNIT |
| 122 | CALCULATOR |
| 123 | ESTIMATOR |
| 125 | RECOMMENDED WATER AMOUNT CALCULATOR |
| 126 | STIMULATION CONTROLLER |
| 127 | RELIABILITY CALCULATOR |
| 130 | OUTPUT CONTROLLER |
| 140 | STORAGE |
| 150 | STIMULUS GENERATOR |
| 150a | COOLING DEVICE |
| 150b | ELECTRODE |
| 200 | BAND UNIT |
| 202 | DISPLAY UNIT |
| 300 | BLOOD FLOW SENSOR |
| 304 | APPLICATION UNIT |
| 306 | DETECTOR |
| 308 | OUTPUT UNIT |
| 400 | MOTION RECOGNITION SENSOR |
| 500 | LEARNING MACHINE |
| 502 | DB |
| 510 | SENSING DATA |
| 512 | LABEL |
| 514 | ESTIMATION RESULT |
| 600 | SPHYGMOMANOMETER |
| 700 | MEASUREMENT AREA |
| 702 | STILL TISSUE |
| 704 | SCATTERING SUBSTANCE |
| 710 | MUSCLE |
| 712 | BLOOD VESSEL |
| 714 | SKIN |
| 800 | INTERFERENCE LIGHT |
| 950 | CPU |
| 952 | ROM |
| 954 | RAM |
| 956 | RECORDING MEDIUM |
| 958 | INPUT-OUTPUT INTERFACE |
| 960 | OPERATION INPUT DEVICE |
| 964 | SOUND OUTPUT DEVICE |
| 962 | DISPLAY DEVICE |
| 968 | COMMUNICATION INTERFACE |
| 970 | BUS |
| 980 | SENSOR |

**Claims**

1. An information processing device comprising a physiological condition estimator configured to estimate a physiological condition of a user who is in a given physical state based on a first blood flow amount change of the user and a second blood flow amount change of the user or another user in the given physical state, the first blood flow amount change and the second blood flow amount change being acquired by a blood flow information detector.

2. The information processing device according to claim 1, wherein the second blood flow amount change is acquired as a reference value previously.

3. The information processing device according to claim 2, further comprising a state detector configured to detect that the user or the another user is in the given physical state by detecting a given motion of the user or the another user in daily motions.

4. The information processing device according to claim 3, wherein the state detector is configured to detect that the user or the another user is in the given physical state based on sensing data that is acquired by the blood flow information detector.

5. The information processing device according to claim 2, further comprising a stimulation controller configured to control a stimulator configured to apply a stimulus that changes a state of a blood vessel of the user or the another user to a body of the user or the another user,
wherein, when the stimulus applied by the stimulator leads to the given physical state, the first blood flow amount change and the second blood flow amount change are acquired by the blood flow information detector.

6. The information processing device according to claim 5, wherein the stimulator is configured to apply the stimulus that cools a skin surface of the user or the another user to the user or the another user.

7. The information processing device according to claim 5, wherein the stimulator is configured to apply the stimulus that squeezes a muscle of the user or the another user to the user or the another user.

8. The information processing device according to claim 5, wherein the stimulator is configured to apply a pressure that blocks a blood flow of the user or the another user.

9. The information processing device according to claim 1, wherein the given physical state is a state in which a blood vessel of the user or the another user is pressurized or a state in which the blood vessel of the user or the another user is contracted.

10. The information processing device according to claim 1, wherein the physiological condition estimator is configured to estimate a future physiological condition of the user based on the first blood flow amount change and the second blood flow amount change.

11. The information processing device according to claim 3, further comprising a context acquisition unit configured to acquire context information on the user or the another user,
wherein the state detector is configured to detect that the user or the another user is in a given physical state based on the context information.

12. The information processing device according to claim 1, wherein a blood flow amount recovery slope is used as the first and second blood flow amount changes, the blood flow amount recovery slope being a slant of an amount of blood flow of the user or the another user with respect to time and being obtained after the amount of blood flow turns to an increase after temporarily decreasing because the user or the another user enters the given physical state.

13. The information processing device according to claim 12, wherein the blood flow recovery slope is a slant that is obtained by dividing a sum of amounts of blood flow until elapse of a given time after the increase of the amount of blood flow by the given time.

14. The information processing device according to claim 12, wherein the physiological condition estimator is configured to estimate that the user has dehydration when a first blood flow amount recovery slope that is the first blood flow amount change is smaller than a reference vale that is obtained from a second blood flow amount recovery slope that is the second blood flow amount change.

15. The information processing device according to claim 14, further comprising a recommended water amount calculator configured to,

when it is estimated that the user has dehydration, estimate an amount of water that is taken by the user based on the first blood flow amount recovery slope and
estimate a recommended water intake amount that is an amount of water that is recommended to take to the user based on the estimated amount of water that is taken.

16. The information processing device according to claim 12, wherein the physiological condition estimator is configured to estimate that the user has anemia when a first blood flow amount recovery slope that is the first blood flow amount change is larger than a reference vale that is obtained from a second blood flow amount recovery slope that is the second blood flow amount change.

17. The information processing device according to claim 14, further comprising reliability calculator configured to calculate a reliability of a result of estimating the physiological state of the user based on a degree of deviation indicating a degree at which the first blood flow amount recovery slope deviates from the second blood flow amount recovery slope.

18. The information processing device according to claim 1, wherein the first blood flow amount change and the second blood flow amount change are acquired by the blood flow information detector that is worn on parts of a body of the user, the parts being different from each other.

19. An Information processing method comprising estimating a physiological condition of a user who is in a given physical state based on a first blood flow amount change of the user and a second blood flow amount change of the user or another user in the given physical state, the first blood flow amount change and the second blood flow amount change being acquired by a blood flow information detector.

20. An program for causing a computer to implement a function of estimating a physiological condition of a user who is in a given physical state based on a first blood flow amount change of the user and a second blood flow amount change of the user or another user in the given physical state, the first blood flow amount change and the second blood flow amount change being acquired by a blood flow information detector.

# FIG.1

200

202

10

# FIG.2

10

300
BLOOD FLOW
SENSOR

100
CONTROLLER

202
DISPLAY
UNIT

400
MOTION
RECOGNITION
SENSOR

# FIG.3

100

ACQUISITION UNIT — 110

CONTEXT ACQUI-SITION UNIT — 111

BLOOD FLOW INFORMATION ACQUISITION UNIT — 112

PROCESSOR — 120

PHYSICAL STATE RECOGNITION UNIT — 121

CALCULATOR — 122

ESTIMATOR — 123

OUTPUT CONTROLLER — 130

STORAGE — 140

# FIG.4

300

CONTROLLER — 302

APPLICATION UNIT — 304

DETECTOR — 306

OUTPUT UNIT — 308

# FIG.5

EP 3 949 842 A1

# FIG.6

# FIG.7

```
        ( START )
            │
   ┌────────┤◄─┐
   │        ▼  │
   │  ┌──────────────────────┐
   │  │ ACQUIRE SENSING DATA FROM │ ～S101
   │  │ MOTION RECOGNITION SENSOR │
   │  └──────────────────────┘
   │        │
   │        ▼
   │  ┌──────────────────────┐
   │  │ DETECT PHYSICAL STATE OF USER │ ～S103
   │  └──────────────────────┘
   │        │
   │        ▼
   │      ╱─────────╲         ～S105
 NO│    ╱ BLOOD FLOW  ╲
◄──┼───╱ AMOUNT         ╲
   │   ╲ DECREASED STATE? ╱
   │    ╲───────────────╱
   │        │YES
   │        ▼
   │  ┌──────────────────────┐
   │  │ ACQUIRE SENSING DATA FROM │ ～S107
   │  │ BLOOD FLOW SENSOR         │
   │  └──────────────────────┘
   │        │
   │        ▼
   │  ┌──────────────────────┐
   │  │ CALCULATE BLOOD FLOW AMOUNT │ ～S109
   │  │ RECOVERY INDEX              │
   │  └──────────────────────┘
   │        │
   │        ▼
   │      ╱─────────╲         ～S111
   │    ╱  OUT OF GIVEN ╲   NO
   └───╲  VALUE RANGE?   ╱──┘
        ╲───────────────╱
            │YES
            ▼
      ┌──────────────────────┐
      │ OUTPUT ESTIMATED         │ ～S113
      │ PHYSIOLOGICAL CONDITION  │
      └──────────────────────┘
            │
            ▼
        ( END )
```

# FIG.8

# FIG.9

# FIG.10

10

# FIG.11

10a

BLOOD FLOW SENSOR — 300

MOTION RECOGNITION SENSOR — 400

CONTROLLER — 100a

DISPLAY UNIT — 202

STIMULUS GENERATOR — 150

# FIG.12

EP 3 949 842 A1

# FIG.13

EP 3 949 842 A1

# FIG.14

100a

**ACQUISITION UNIT** — 110

- CONTEXT ACQUI-SITION UNIT — 111
- BLOOD FLOW INFORMATION ACQUISITION UNIT — 112

**PROCESSOR** — 120a

- PHYSICAL STATE RECOGNITION UNIT — 121
- CALCULATOR — 122
- ESTIMATOR — 123
- STIMULATION CONTROLLER — 126

**OUTPUT CONTROLLER** — 130

**STORAGE** — 140

# FIG.15

START

GENERATE STIMULUS — S201

ACQUIRE SENSING DATA FROM BLOOD FLOW SENSOR — S203

CALCULATE BLOOD FLOW AMOUNT RECOVERY INDEX — S205

OUT OF GIVEN VALUE RANGE? — S207

NO

YES

OUTPUT ESTIMATED PHYSIOLOGICAL CONDITION — S209

END

# FIG.16

10b

600

300
BLOOD FLOW SENSOR

100
CONTROLLER

202
DISPLAY UNIT

SPHYGMO-MANOMETER

# FIG.17

# FIG.18

# FIG.19

```
┌─────────┐510        ┌─────────┐123        ┌──────────┐514
│SENSING  │    →      │ESTIMATOR│    →      │ESTIMATION│
│DATA     │           │         │           │RESULT    │
└─────────┘           └─────────┘           └──────────┘
                          ↑
                      ┌───────┐
                      │  DB   │ 502
                      └───────┘
```

# FIG.20

100b

```
                    110              120b              130
┌──────────────────────────────────────────────────────────────────────┐
│  ┌─────────────────┐    ┌─────────────────┐   ┌──────────────┐        │
│  │ACQUISITION UNIT │    │   PROCESSOR     │   │OUTPUT        │        │
│  │          ∿111   │    │          ∿121   │   │CONTROLLER    │        │
│  │┌───────────────┐│    │┌───────────────┐│   └──────────────┘        │
│  ││CONTEXT ACQUI- ││    ││PHYSICAL STATE ││              140          │
│  ││SITION UNIT    ││ →  ││RECOGNITION    ││   ┌──────────────┐        │
│  │└───────────────┘│    ││UNIT           ││   │   STORAGE    │        │
│  │          ∿112   │    │└───────────────┘│ ↔ └──────────────┘        │
│  │┌───────────────┐│    │          ∿122   │                          │
│  ││BLOOD FLOW     ││    │┌───────────────┐│                          │
│  ││INFORMATION    ││    ││CALCULATOR     ││                          │
│  ││ACQUISITION UNIT││   │└───────────────┘│                          │
│  │└───────────────┘│    │          ∿123   │                          │
│  └─────────────────┘    │┌───────────────┐│                          │
│                         ││ESTIMATOR      ││                          │
│                         │└───────────────┘│                          │
│                         │          ∿125   │                          │
│                         │┌───────────────┐│                          │
│                         ││RECOMMENDED    ││                          │
│                         ││WATER AMOUNT   ││                          │
│                         ││CALCULATOR     ││                          │
│                         │└───────────────┘│                          │
│                         └─────────────────┘                          │
└──────────────────────────────────────────────────────────────────────┘
```

# FIG.21

```
        ┌─────────┐
        │  START  │
        └─────────┘
             │
             ▼
┌─────────────────────────────┐
│ ACQUIRE SENSING DATA FROM   │── S301
│ MOTION RECOGNITION SENSOR   │
└─────────────────────────────┘
             │
             ▼
┌─────────────────────────────┐
│  DETECT PHYSICAL STATE OF   │── S303
│          USER               │
└─────────────────────────────┘
             │
             ▼
         ╱─────────╲
        ╱ BLOOD FLOW ╲── S305
       ╱ AMOUNT DECREASED ╲ ── NO
       ╲    STATE?    ╱
        ╲───────────╱
           │ YES
           ▼
┌─────────────────────────────┐
│ ACQUIRE SENSING DATA FROM   │── S307
│     BLOOD FLOW SENSOR       │
└─────────────────────────────┘
             │
             ▼
┌─────────────────────────────┐
│   CALCULATE BLOOD FLOW      │── S309
│  AMOUNT RECOVERY INDEX      │
└─────────────────────────────┘
             │
             ▼
┌─────────────────────────────┐
│   CALCULATE RECOMMENDED     │── S311
│       WATER AMOUNT          │
└─────────────────────────────┘
             │
             ▼
┌─────────────────────────────┐
│    OUTPUT RECOMMENDED       │── S313
│       WATER AMOUNT          │
└─────────────────────────────┘
             │
             ▼
        ┌─────────┐
        │   END   │
        └─────────┘
```

# FIG.22

# FIG.23

# FIG.24

START

ACQUIRE SENSING DATA FROM MOTION RECOGNITION SENSOR — S401

DETECT PHYSICAL STATE OF USER — S403

BLOOD FLOW AMOUNT DECREASED STATE? — S405

NO

YES

ACQUIRE SENSING DATA FROM BLOOD FLOW SENSOR — S407

CALCULATE BLOOD FLOW AMOUNT RECOVERY INDEX — S409

OUT OF GIVEN VALUE RANGE? — S411

NO

YES

OUTPUT ESTIMATED PHYSIOLOGICAL CONDITION — S413

OUTPUT RELIABILITY — S415

END

# FIG.25

DEHYDRATION.
RELIABILITY IS
DD%.

10

# FIG.26

LIFT HAND

AMOUNT OF BLOOD FLOW (LIFTED HAND)

TIME

AMOUNT OF BLOOD FLOW (NOT-LIFTED HAND)

TIME

# FIG.27

900

- CPU 950
- ROM 952
- RAM 954
- RECORDING MEDIUM 956
- 970
- INPUT-OUTPUT INTERFACE 958
- COMMUNICATION INTERFACE 968
- SENSOR 980
- OPERATION INPUT DEVICE 960
- DISPLAY DEVICE 962
- SOUND OUTPUT DEVICE 964

**INTERNATIONAL SEARCH REPORT**

International application No.

PCT/JP2020/011711

A. CLASSIFICATION OF SUBJECT MATTER
Int. Cl. A61B5/0285(2006.01)i
FI: A61B5/0285 H

According to International Patent Classification (IPC) or to both national classification and IPC

B. FIELDS SEARCHED

Minimum documentation searched (classification system followed by classification symbols)
Int. Cl. A61B5/0285

Documentation searched other than minimum documentation to the extent that such documents are included in the fields searched
Published examined utility model applications of Japan     1922-1996
Published unexamined utility model applications of Japan   1971-2020
Registered utility model specifications of Japan           1996-2020
Published registered utility model applications of Japan   1994-2020

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2015-54219 A (CASIO COMPUTER CO., LTD.) 23 March 2015, paragraphs [0010]-[0078] | 1-4, 10, 12-15, 19-20 |
| Y | | 5-9, 11, 16, 18 |
| A | | 17 |
| Y | JP 2002-172095 A (K & S KK) 18 June 2002, paragraphs [0001]-[0041] | 5-9, 18 |
| Y | US 2013/0324866 A1 (GLADSHTEIN, Reuven) 05 December 2013, paragraphs [0068]-[0168] | 5-9 |
| Y | JP 2018-108278 A (KYOCERA CORP.) 12 July 2018, paragraphs [0015]-[0213] | 11 |
| A | | 17 |

☒ Further documents are listed in the continuation of Box C.   ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 28.05.2020 | 09.06.2020 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

## INTERNATIONAL SEARCH REPORT

International application No.

PCT/JP2020/011711

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 11-76216 A (SHIMADZU CORP.) 23 March 1999, paragraphs [0001]-[0041] | 16 |

Form PCT/ISA/210 (continuation of second sheet) (January 2015)

**EP 3 949 842 A1**

| Patent Documents referred to in the Report | Publication Date | Patent Family | Publication Date |
|---|---|---|---|
| JP 2015-54219 A | 23.03.2015 | (Family: none) | |
| JP 2002-172095 A | 18.06.2002 | US 2002/0095092 A1 paragraphs [0025]-[0110] EP 1212979 A2 | |
| US 2013/0324866 A1 | 05.12.2013 | WO 2012/110955 A1 | |
| JP 2018-108278 A | 12.07.2018 | (Family: none) | |
| JP 11-76216 A | 23.03.1999 | (Family: none) | |

Form PCT/ISA/210 (patent family annex) (January 2015)

**EP 3 949 842 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015054219 A